# EUROPÄISCHE PATENTSCHRIFT

(11) **EP 3 135 273 B1**
(45) Veröffentlichungstag und Bekanntmachung des Hinweises auf die Patenterteilung: **15.07.2020**
(21) Anmeldenummer: 15182351.5
(22) Anmeldetag: 25.08.2015
(51) Int. Cl.: A61K 31/194, A61K 9/00, A61K 8/00, A61K 33/06, A61K 33/30, A61P 3/04, A61P 3/10, A61P 43/00, A61P 3/00, A61K 9/16

(54) **MINERALSTOFFZUSAMMENSETZUNGEN ZUR ANREGUNG DES KOHLENHYDRATSTOFFWECHSELS**
MINERAL COMPOSITIONS FOR STIMULATING CARBOHYDRATE METABOLISM
COMPOSITIONS DE MATIERES MINERALES DESTINEES A AMORCER LE METABOLISME GLUCIDIQUE

(43) Veröffentlichungstag der Anmeldung: 01.03.2017
(73) Patentinhaber: Protina Pharmazeutische Gesellschaft mbH, 85737 Ismaning (DE)
(72) Erfinder: Werner, Tanja, 85579 Neubiberg (DE)
(74) Vertreter: Eisenführ Speiser

(56) Entgegenhaltungen:
- EP-A1- 2 072 046
- WO-A1-02/102362
- WO-A1-2012/011063
- US-A1- 2003 099 626
- US-A1- 2008 193 525
- US-A1- 2008 206 412
- US-A1- 2008 249 169
- Anonymous: "Basica Direkt", , 25. April 2015 (2015-04-25), XP055241863, Gefunden im Internet: URL:http://web.archive.org/web/20150425024 701/http://www.basica.info/products/basica -direkt/ [gefunden am 2016-01-15]
- MEGAHED MAGDA A GHANY ET AL: "Effect of oral magnesium supplementation on blood magnesium, fasting blood glucose, glycosylated hemoglobin levels and lipid pattern in type II hypomagnesmic diabetes mellitus patients with complications", JOURNAL OF THE MEDICAL RESEARCH INSTITUTE, ALEXANDRIA UNIVERSITY, ALEXANDRIA, EG, Bd. 24, Nr. 2, 1. Januar 2003 (2003-01-01) , Seiten 79-89, XP008179973, ISSN: 1110-0133
- P.-C. T. PHAM ET AL: "Hypomagnesemia in Patients with Type 2 Diabetes", CLINICAL JOURNAL OF THE AMERICAN SOCIETY OF NEPHROLOGY, Bd. 2, Nr. 2, 14. Februar 2007 (2007-02-14), Seiten 366-373, XP055266965, ISSN: 1555-9041, DOI: 10.2215/CJN.02960906

## Beschreibung

### Technisches Gebiet

Die vorliegende Erfindung betrifft eine Mischung aus drei oder mehr Citratsalzen, umfassend Magnesiumcitrat, wobei die Mischung aus drei oder mehr Citratsalzen zusätzlich Calciumcitrat und/oder Zinkcitrat umfasst, zur Anwendung in einem therapeutischen Verfahren gemäß den Ansprüchen.

Ein weiterer Aspekt der vorliegenden Erfindung betrifft eine Zusammensetzung zur Anwendung in einem therapeutischen Verfahren gemäß den Ansprüchen, wobei die Zusammensetzung eine Mischung aus drei oder mehr Citratsalzen, umfassend Magnesiumcitrat, als aktiven Wirkstoff enthält, wobei die Mischung aus drei oder mehr Citratsalzen zusätzlich Calciumcitrat und/oder Zinkcitrat umfasst.

Des Weiteren betrifft die vorliegende Erfindung auch die nicht-therapeutische Verwendung einer Mischung aus drei oder mehr Citratsalzen wie hierin beschrieben oder einer Zusammensetzung wie hierin beschrieben, zum Erreichen einer oder mehrerer nichttherapeutischer/therapeutischen Wirkung(en).

Weitere Aspekte der vorliegenden Erfindung und bevorzugte Ausgestaltungen davon ergeben sich aus der nachfolgenden Beschreibung, den Ausführungsbeispielen und den Ansprüchen.

### Hintergrund der Erfindung

Der Stoffwechsel, auch Metabolismus genannt, steht für alle biochemischen Vorgänge im Körper, die innerhalb der Zellen ablaufen und ist die Grundlage aller lebenswichtigen Vorgänge im Körper. Die Bestandteile der zugefügten Makronährstoffe in der Nahrung, umfassend Kohlenhydrate, Fette, Proteine und Mineralstoffe, werden im Magen und Darm in ihre Bestandteile zerlegt. Kohlenhydrate werden umgewandelt in Monosaccharide, Proteine in Aminosäuren und Fette in Fettsäuren und Glyceride, da der Darm die Nährstoffe nur in ihrer zerlegten Form resorbieren kann.

Es gibt verschiedene Arten des Stoffwechsels, den Kohlenhydrat-, Eiweiß-, Fett- und Mineralstoffwechsel, die nach den Substanzen, die dabei verarbeitet werden, benannt sind.

Beim Kohlenhydratstoffwechsel gelangen die in der Verdauung gewonnenen Monosaccharide über das Blut in die Zellen. Wenn gerade keine Energie benötigt wird, wird das Monosaccharid Glukose mit Hilfe von Insulin in der sogenannten Glykogensynthese als Glykogen gespeichert und bei späterem Energiebedarf wird durch Glukagon oder Adrenalin der Abbau von Glykogen aktiviert (E. Buddecke, Grundriss der Biochemie, Walter de Gruyter Verlag, 6. Auflage, 1980).

Direkt benötigte Energie wird durch den schrittweisen Abbau der aus der Nahrung oder dem Glykogenabbau gewonnen Monosaccharide im Zytoplasma der Zellen gewonnen. Bei dieser sogenannten Glykolyse entstehen aus einem Glukosemolekül unter anderem zwei Moleküle Pyruvat, wobei ein besonders hoher Energiegewinn erzielt wird. Zum einen entsteht Energie direkt in der Form von ATP, zum anderen indirekt als NADH/H⁺, aus dem dann in der mitochondrialen Atmungskette ATP erzeugt wird. Der Abbau von Glukose bis zu Pyruvat läuft dabei sowohl unter anaeroben als auch unter aeroben Bedingungen gleichartig ab.

Bei ausreichendem Sauerstoffangebot erfolgt in den Mitochondrien anschließend mit Hilfe der Pyruvatdehydrogenase die Umwandlung von Pyruvat zu Acetyl-CoA, welches über den Citratzyklus weiter zu CO₂ und Wasser oxidiert wird und damit direkt am zellulären Energiestoffwechsel beteiligt ist. Bei Sauerstoffmangel hingegen wird Pyruvat im Zytosol hauptsächlich zu Lactat reduziert (G. Löffler G., P. E. Petrides, P. C. Heinrich, Biochemie & Pathobiochemie 2007, 8. Auflage, Springer Medizin Verlag Heidelberg). Das Lactat kann in Säugetierzellen nicht weiter verstoffwechselt werden, sondern nur, falls in ausreichender Menge NAD zur Verfügung steht, zurück in Pyruvat umgewandelt werden. Daher wird es auch als "Stoffwechselsackgasse" bezeichnet (H. R. Horton, L. A. Moran, K. G. Scrimgeour, M. D. Perry, J. D. Rawn, Biochemie, Pearson Studium, 4. aktualisierte Auflage, 2008).

Bei stark erhöhter körperlicher Belastung ist die Glykolyse zur zusätzlichen Energiegewinnung um ein Vielfaches gesteigert. Dies hat eine erhöhte Pyruvat-Synthese zur Folge, die im Citratzyklus jedoch nicht vollständig verarbeitet werden kann. Das überschüssige Pyruvat wird zu Lactat umgewandelt.

Eine erhöhte Lactatkonzentration kann also die Folge starker muskulärer Belastungen oder auch von Erkrankungen sein, die mit einem herabgesetzten Sauerstoffangebot einhergehen. Dies sind vor allem Infektionen, Durchblutungsstörungen, Stoffwechselstörungen (insbesondere Diabetes mellitus) sowie Stress-Situationen (oxidativer und Nitrostress). Sind die Energiequellen erschöpft, werden die Energiereserven der Kohlenhydrate mobilisiert. Der gesteigerte Glukoseabbau hat eine erhöhte Pyruvat-Bildung zur Folge. Das in Übermaß entstandene Pyruvat wird, auch in Folge des erniedrigten Sauerstoffdruckes, überwiegend zu Lactat reduziert.

Zudem können mitochondriale Erkrankungen, bei denen der zelluläre Energiestoffwechsel beeinträchtigt ist, zu einer erhöhten Lactatkonzentration führen. Ein erhöhter Lactatwert findet sich zum Beispiel bei einem Defekt der Pyruvatoxidation. Durch Anstauung des Metaboliten Pyruvat wird vermehrt Lactat gebildet. Auch bei Atmungskettendefizienzen findet sich eine Verschiebung des Gleichgewichts der Lactatdehydrogenase-Reaktion von Pyruvat in Richtung Lactat (H. Renz, Integrative klinische Chemie und Laboratoriumsmedizin, Walter de Gruyter GmbH&Co., 2003).

Neben Lactat entstehen H+-lonen zur Abpufferung. Hieraus resultiert eine pH-Verschiebung in den sauren Bereich, die erhebliche Störungen des Stoffwechsels, insbesondere Aktivitätseinschränkungen der Enzyme, die sogenannte Lactat-Azidose, nach sich ziehen kann.

Die entstandene Lactat-Azidose setzt einen Circulus vitiosus in Gang: Durch die beeinträchtigte Pyruvat-Dehydrogenase ist die Acetyl-CoA-Bildung aus Pyruvat blockiert. Acetyl-CoA wird normalerweise im Citratzyklus metabolisiert. Hierbei entstehen die Reduktionsäquivalente NADH und FADH₂, die in der Atmungskette zur Herstellung von ATP genutzt werden. Steht Acetyl-CoA jedoch nicht mehr in ausreichendem Maße als Ausgangspunkt für den Citratzyklus zur Verfügung, führt dies zu einer verminderten Bereitstellung der Redoxsubstanzen NADH und FADH₂. Die Folge hiervon ist eine starke Drosselung der Energieproduktion und Beeinträchtigung der Mitochondrienfunktion. Das herabgesetzte Energieangebot betrifft vor allem Zellen mit einem hohen Energiebedarf wie Nervenzellen, Muskulatur, Herzmuskel und Zellen des Immunsystems.

Als mögliche Ursachen für einen erhöhten Lactat-Spiegel können außerdem auch Medikamente wie z. B. Statine, Metformin oder Isoniazid in Betracht kommen.

Die Symptome eines vorliegenden Lactat-Überschusses umfassen je nach Schweregrad Lethargie, Erbrechen, Bauchschmerzen, Appetitlosigkeit, Dehydration, Hyperventilation, Hypotension, Zyanose, Tachykardie, kardiovaskuläre Erkrankungen, Unruhe, Verwirrtheit, Müdigkeit und Bewusstseinseintrübung bis hin zum Koma.

Störungen des Kohlenhydratstoffwechsels, wie sie z.B. bei durch mitochondriale Erkrankungen erzeugten Störungen des zellulären Energiestoffwechsels vorliegen können, können zudem durch die Ermittlung der Lactat/Pyruvat-Ratio analytisch erfasst werden. Das Verhältnis der Lactat- und Pyruvatkonzentrationen im Blutplasma oder Urin zeigt an, ob aerobe oder anaerobe Stoffwechselprozesse im Körper überwiegen. Durch die Verwendung von Mikrodialyse-Methoden können auch *in vivo* Untersuchungen des Stoffwechsels einfach zugänglicher Gewebe wie z.B. der Skelettmuskeln erfolgen. Die Bestimmung der Lactat/Pyruvat-Ratio im Dialysat ermöglicht in diesem Fall, das Verhältnis von anaerober zu aerober Stoffwechsellage im Muskel zu beurteilen. Eine Lactat/Pyruvat-Ratio von über 10 bis 20 zeigt generell einen verschlechterten Kohlenhydratstoffwechsel und damit eine verschlechterte zelluläre Energiebilanz, z.B. aufgrund einer unzureichenden Sauerstoffzufuhr oder mitochondrialen Dysfunktion, an.

Die Therapie eines erhöhten Lactat-Spiegels bzw. einer akuten Lactat-Azidose kann, je nach Schweregrad, die Gabe von Coenzym Q10, L-Carnitin, Alpha-Liponsäure, Biotin sowie der Vitamine B1, B2, B3, B5, die Kreislaufstabilisierung durch kreislaufunterstützende Medikamente, Beendigung einer eventuell bestehenden Metformin-Therapie, Normalisierung des Blutzuckerspiegels durch Insulingabe und unter Umständen die Durchführung einer Dialyse zur Entfernung des Lactats aus dem Körper umfassen.

Ein Nachteil der Supplementation von Coenzym Q10 ist jedoch, dass es die Blutgerinnung fördert. Daher sollten Personen, die blutgerinnungshemmende Mittel einnehmen, auf Q10 als Nahrungsergänzungsmittel verzichten oder zumindest Rücksprache mit dem Arzt halten. L-Carnitin dient unter anderem auch als Motor für die Schweißproduktion, daher verliert der Körper insbesondere bei einer zu starken Dosierung zu viel Wasser, was durch eine verstärkte Wasseraufnahme ausgeglichen werden muss. Die Gabe von Alpha-Liponsäure kann in seltenen Fällen zu Übelkeit, Erbrechen, Magen-, Darmschmerzen, Durchfall, allergischen Reaktionen wie Hautausschlag, Nesselsucht (Urtikaria) und Juckreiz und zur Veränderung bzw. Störung des Geschmacksempfindens führen. Biotin ist in der Regel gut verträglich, sehr selten wurden jedoch allergische Reaktionen der Haut (Urtikaria) beschrieben. Es bestehen zudem Hinweise, dass die gleichzeitige Gabe von Arzneimitteln gegen Krampfanfälle (Antikonvulsiva) den Spiegel von Biotin im Blutplasma senkt.

Vitamin-B-Komplex-Präparate standen in den letzten Jahren teilweise in der Kritik, viel zu hoch dosiert zu sein. So hat etwa die Stiftung Ökotest entsprechende Präparate negativ bewertet, wenn die Dosierungen deutlich über den offiziellen Empfehlungen lagen. Zudem ergab die zweite nationale Verzehrsstudie des Bundesministeriums für Ernährung, Landwirtschaft und Verbraucherschutz, dass die Bundesbürger durch die Nahrung mit Ausnahme von Folsäure mit B-Vitaminen bereits gut versorgt sind (ÖKO-TEST Jahrbuch Gesundheit für 2010). So kann, zum Beispiel, die Supplementation von größeren Mengen an Nicotinsäure (Vitamin B3) schwerwiegende gesundheitliche Effekte auslösen, wie z.B. gastrointestinale unerwünschte Wirkungen, Glukoseintoleranz sowie hepatotoxische Wirkungen (Bundesinstitut für Risikobewertung, Stellungnahme Nr. 018/2012 vom 06. Februar 2012). Vitamin B2 färbt zudem den Urin neongelb, was von einigen Patienten als unangenehm empfunden werden kann. WO 2012/011063 A1 offenbart Polydeoxyribonucleotide (PDRNs) zur Behandlung von Lactat-Azidose. WO 02/102362 A1 offenbart Zitronensäure zur Behandlung von Adipositas.

### Zusammenfassung der Erfindung

Im Hinblick auf die oben genannten negativen Auswirkungen eines ineffektiven Kohlenhydratstoffwechsels auf den Organismus besteht ein ständiges Bedürfnis, Stoffe bzw. Zusammensetzungen bereitzustellen, die den Kohlenhydratstoffwechsel, insbesondere den oxidativen Glukose-Stoffwechsel und dadurch den zellulären Energiestoffwechsel, anregen oder verstärken.

Primäre Aufgabe der vorliegenden Erfindung war es daher, solche Stoffe bzw. Zusammensetzungen bereitzustellen.

Ziel der vorliegenden Erfindung war es zudem, molekulare Mechanismen zur erforschen, welche den Kohlenhydratstoffwechsel beeinflussen und damit mögliche förderliche Wirkung auf ubiquitäre Stoffwechselerkrankungen wie Diabetes mellitus, Adipositas oder das metabolische Syndrom haben, idealerweise solche, welche zudem einen möglichen förderlichen Einfluss auf den Kohlenhydratstoffwechsel und den Säuren-Basen-Haushalt haben.

Vorzugsweise sollen die bereitgestellten Stoffe bzw. Zusammensetzungen eine gute Bioverfügbarkeit aufweisen, um den/die aktiven Wirkstoff(e) einem Individuum in geeigneter Weise zuzuführen, sodass eine optimale Versorgung mit dem/den aktiven Wirkstoff(en) erzielt wird.

Die Erfindung betrifft eine Mischung aus drei oder mehr Citratsalzen, umfassend Magnesiumcitrat, wobei die Mischung aus drei oder mehr Citratsalzen zusätzlich Calciumcitrat und/oder Zinkcitrat umfasst, zur Anwendung in einem therapeutischen Verfahren zum Vorbeugen und/oder Behandeln einer Lactat-Azidose und/oder von Adipositas.

Zudem betrifft die Erfindung eine Zusammensetzung zur Anwendung in einem therapeutischen Verfahren, wobei die Zusammensetzung eine Mischung aus drei oder mehr Citratsalzen, umfassend Magnesiumcitrat, als aktiven Wirkstoff enthält, wobei die Mischung aus drei oder mehr Citratsalzen zusätzlich zum Magnesiumcitrat zwei oder mehr Citratsalze aus der Gruppe bestehend aus Calciumcitrat, Zinkcitrat, Natriumcitrat, Kupfercitrat, Eisencitrat, Kaliumcitrat, Kalium-Natrium-Hydrogencitrat, Calcium-Natrium-Hydrogencitrat, di-Natriumhydrogencitrat, Ammoniumcitrat, Ammoniumhydrogencitrat, Molybdäncitrat, Mangancitrat, Lithiumcitrat und Chromcitrat, umfasst, wobei das therapeutische Verfahren ein Verfahren zum Vorbeugen und/oder Behandeln einer Lactat-Azidose und/oder von Adipositas ist.

Des weiteren betrifft die Erfindung die nicht-therapeutische Verwendung einer Mischung aus drei oder mehr Citratsalzen wie oben definiert oder einer Zusammensetzung wie oben definiert, zum Erreichen einer nicht-therapeutischen Wirkung ausgewählt aus der Gruppe bestehend aus: Verhinderung eines nicht-pathologischen Lactatüberschusses im Muskel durch das Anregen oder Verstärken des Kohlenhydratstoffwechsels, Förderung der Verdauung und einer unterstützenden Wirkung bei der Gewichtsreduktion.

### Definitionen

Der Begriff Citratsalz, wie hierin verwendet, bezeichnet Salze der Citronensäure, d.h. Verbindungen umfassend Citrate, Hydrogencitrate und Dihydrogencitrate als Anionen mit ein, zwei oder drei Kationen als Gegenionen. Auch Hydrate der Citratsalze gelten als im Rahmen der vorliegenden Offenbarung unter dem Begriff Citratsalz mit umfasst.

Der Begriff Proband(in), wie hierin verwendet, bezieht sich auf eine Person, an der ein wissenschaftlicher Versuch oder Test durchgeführt wird.

Der Begriff Placebo, wie hierin verwendet, bezeichnet eine Scheinintervention, d.h. im Rahmen der vorliegenden Offenbarung die Supplementation von Zusammensetzungen, die keine pharmakologische Wirkung haben können.

Der Begriff Verum, wie hierin verwendet, bezeichnet im Rahmen der vorliegenden Offenbarung die Supplementation von Zusammensetzungen, die im Gegensatz zum wirkstofffreien Placebo pharmakologisch wirksame Substanzen enthalten.

Der Begriff Interventionsstudie, wie hierin verwendet, bezieht sich auf eine experimentelle Studie in der Epidemiologie, bei der der Untersucher durch bewusste Manipulation kausaler Faktoren (Intervention) deren Einfluss auf einen Endpunkt prüft, um damit eine a *priori* aufgestellte Hypothese zu bestätigen bzw. zu widerlegen. Im Gegensatz zur Beobachtungsstudie handelt es sich um ein Experiment, mit dem die Effektivität einer bestimmten Intervention, in der vorliegenden Offenbarung eine Supplementation von Zusammensetzungen enthaltend Citratsalze, untersucht werden soll. Im einfachsten Fall bildet der Untersucher zwei Gruppen: Bei der Interventionsgruppe (Verum-Gruppe) wird die zu untersuchende Intervention durchgeführt, bei der Kontrollgruppe (Placebo-Gruppe) unterbleibt sie.

Der Begriff randomisierte Interventionsstudie, wie hierin verwendet, bezeichnet eine Interventionsstudie, bei der die Zuordnung der Probanden zur Interventions- oder Kontrollgruppe zufällig erfolgt. Dadurch ist gewährleistet, dass beide Gruppen in ihrer Zusammensetzung zueinander weitgehend äquivalent sind. In der vorliegenden Offenbarung erfolgte die Randomisierung durch eine nicht an der Durchführung der Studie beteiligte Person. Dazu wurden nach schriftlicher Einwilligungserklärung aus einer zuvor angefertigten Serie verschlossener Umschläge mit fortlaufender Patientennummer die Gruppen-Zuordnungen ermittelt.

Der Begriff doppelblinde Interventionsstudie, wie hierin verwendet, bezeichnet eine Interventionsstudie, bei der weder der Versuchsleiter (Arzt) noch die Studienteilnehmer (Probanden) Kenntnis über die jeweilige Gruppenzugehörigkeit (Interventionsgruppe oder Kontrollgruppe) haben, d.h. weder der Arzt noch der Patient wissen wer das wirkstoffhaltige Interventionsmittel und wer das Placebo erhält. Dies dient der Verhinderung einer Verzerrung der Studienergebnisse.

Der Begriff postrandial, wie hierin verwendet, steht für "nach dem Essen" bzw. "nach einer Testmahlzeit".

Der Begriff Mikrodialyse, wie hierin verwendet, bezeichnet eine einfache, minimalinvasive Methode, die auf dem Prinzip der Dialyse beruht. Sie erlaubt *in vivo* Untersuchungen zur Regulation von Durchblutung und Stoffwechsel einfach zugänglicher Gewebe wie dem subkutanen Fettgewebe oder dem Skelettmuskel am Menschen. Die in das Gewebe eingeführte Mikrodialyse-Sonde ist doppel-lumig, bestehend aus einem äußeren, zuführenden und einem inneren, abführenden Tubus. Der äußere Tubus ist in seinem distalen Teil über eine bestimmte Distanz semipermeabel. Die Sonde wird normalerweise mit einer geringen Flussgeschwindigkeit mit beispielsweise einer physiologischen Salzlösung (Perfusat) durchströmt. Das Perfusat fließt über den äußeren Tubus entlang an der semipermeablen Membran vorbei, gelangt über eine kleine Öffnung am distalen Teil in den inneren Tubus, über den es die Sonde wieder verlässt und in speziellen Mikrogefäßen (Mikrovials) fraktioniert gesammelt werden kann. An der semipermeablen Membran können abhängig von den chemischen und molekularen Eigenschaften der Membran und der verschiedenen Komponenten des Interstitiums unterschiedliche Moleküle entsprechend des Konzentrationsgefälles durch die Membran in das Perfusat treten, wodurch dieses zum Dialysat wird. Da die Sonde kontinuierlich perfundiert wird, kann sich zwischen dem Interstitium und dem Perfusat kein Konzentrationsgleichgewicht einstellen. Die Konzentration eines Moleküls im Interstitium ist jedoch direkt proportional zu seiner Konzentration im Dialysat. Das Ausmaß der Diffusion von Substanzen aus dem Interstitium in das Perfusat, die so genannte Wiederfindungsrate im Dialysat (Recovery), hängt von mehreren Faktoren ab. Zum einen spielt die Perfusionsgeschwindigkeit und die Länge der Membran eine wichtige Rolle. Eine lange Mikrodialyse-Sonde kombiniert mit einer langsamen Perfusionsrate führen zu einer verbesserten Recovery. Wird eine 30 mm lange Membran mit einer Geschwindigkeit von 0,3 µl/min perfundiert, beträgt die Recovery nahezu 100%. Weitere, die Recovery beeinflussende Faktoren sind Molekulargewicht sowie physikalische und chemische Eigenschaften (z.B. Ladung und Löslichkeit) des zu untersuchenden Moleküls (P. Arner, Proc. Nutr. Soc. 1999, 58/04, 913-917). Die Porengröße der verwendeten Membran (molekulares Cut-off) limitiert zudem die Größe der Moleküle, die sie passieren können.

Der Begriff Dialysat, wie hierin verwendet, bezeichnet die nach dem Mikrodialyse-Schritt gesammelten Perfusat-Proben.

Der Begriff EtOH-Ratio (Ethanol-Ratio), wie hierin verwendet, bezeichnet den Quotienten aus der Ethanolkonzentration im Dialysat und der Ethanolkonzentration im Perfusat. Durch die EtOH-Ratio lässt sich die Durchblutung im Gewebe semiquantitativ beurteilen. Dafür wird eine definierte Menge an Ethanol zum Perfusat gegeben. Ethanol diffundiert aus dem Perfusat entsprechend seines Konzentrationsgradienten in das Interstitium und wird über die Blutkapillaren, je nach der Stärke der Gewebedurchblutung schneller oder langsamer, abtransportiert. Demnach ist die Ethanol-Konzentration im Dialysat stets niedriger als im Perfusat und kann zur semiquantitativen Analyse der Gewebedurchblutung herangezogen werden..

Der Energieumsatz (EU), wie hierin verwendet, setzt sich zusammen aus dem Grundumsatz (GU) und dem Leistungsumsatz. Der GU ist die Energiemenge, die für die Aufrechterhaltung aller grundlegenden, lebenserhaltenden Körperfunktionen und des Muskeltonus nötig ist. Der GU wird morgens, direkt nach dem Aufwachen im Liegen, 12-14 h nach Einnahme der letzten Mahlzeit bei angenehmer Zimmertemperatur gemessen. Der Leistungsumsatz ist die Energiemenge, die der Körper innerhalb eines Tages benötigt, um Arbeit zu verrichten. Als Leistungsumsatz wird dabei die Energie bezeichnet, die über den Grundumsatz hinausgeht. Der Ruhe-Nüchtern-Umsatz (RNU), wie hierin verwendet, bezeichnet den Energieumsatz, der im Gegensatz zum im Liegen gemessenen Grundumsatz, im Sitzen bestimmt wird und etwa 10% über dem Grundumsatz liegt (G. Püschel, H. Kühn, T. Kiethmann, Taschenlehrbuch der Biochemie 2011; Georg Thieme Verlag KG, Stuttgart).

Der Begriff postprandiale Thermogenese (ppTh), wie hierin verwendet, bezeichnet die Steigerung des Energieumsatzes nach der Nahrungsaufnahme. Sie resultiert aus der für Verdauung, Resorption, Transport und Speicherung der Nährstoffe benötigten Energie. Die ppTh hängt ab von Zusammensetzung und Menge der aufgenommenen Nahrung und führt zu gleichen Umsatzsteigerungen unabhängig von Alter und Geschlecht. Für die einzelnen Makronährstoffe werden folgende Umsatzsteigerungen gemessen: Fette 2-4%, Kohlenhydrate 4-7% und Proteine 18-25% (H. K. Biesalski, P. Fürst, H. Kasper, Ernährungsmedizin: Nach dem Curriculum Ernährungsmedizin der Bundesärztekammer. 2004, 3. erweiterte Auflage, Georg Thieme Verlag, Stuttgart). Proteine haben somit das höchste thermogene Potential, gefolgt von Kohlenhydraten und Fetten. Bezogen auf den 24h Gesamtenergieumsatz liegt der Anteil des RNU bei ca. 60-70%, der der ppTh bei ca. 5-10%, und der der körperlichen Aktivität bei 20-30%.

Der Begriff Kohlenhydratoxidationsrate (KHO), wie hierin verwendet, bezeichnet die Rate mit welcher die Kohlenhydrate oxidativ unter der Bildung von Kohlenstoffdioxid verbrannt werden.

Der Begriff oxidativer Glukose-Stoffwechsel, wie hierin verwendet, bezieht sich, wie oben ausführlich beschrieben, auf den Abbau des aus der Nahrung gewonnenen Monosaccharids Glukose unter aerober Stoffwechsellage. Der erste Teil des Glukose-Stoffwechsels ist die Glykolyse - ein biochemischer Abbauweg, der ein Molekül Glukose in zwei Moleküle Pyruvat umwandelt und unter anaeroben und aeroben Bedingungen gleichartig abläuft. Aerob arbeitende Gewebe bauen das Pyruvat anschließend im Citratzyklus und der sich anschließenden Atmungskette unter Energiegewinnung weiter zu den Endprodukten Kohlenstoffdioxid und Wasser ab.

### Detaillierte Beschreibung

Gegenstände, die nicht durch den Schutzumfang der Ansprüche umfasst sind, sind nicht Teil der beanspruchten Erfindung. Ein primärer Aspekt der vorliegenden Offenbarung betrifft ein Citratsalz, nämlich ein Magnesiumcitrat, oder eine Mischung aus zwei, drei oder mehr Citratsalzen, umfassend Magnesiumcitrat, wobei die Mischung aus zwei, drei oder mehr Citratsalzen zusätzlich Calciumcitrat und/oder Zinkcitrat umfasst, zur Anwendung in einem therapeutischen Verfahren zum Anregen oder Verstärken des Kohlenhydratstoffwechsels, vorzugsweise des Glukose-Stoffwechsels, insbesondere zum Anregen oder Verstärken des zellulären Energiestoffwechsels.

Ein Aspekt betrifft daher das Citratsalz Magnesiumcitrat zur Anwendung in einem therapeutischen Verfahren zum Anregen oder Verstärken des Kohlenhydratstoffwechsels, vorzugsweise des Glukose-Stoffwechsels, insbesondere zum Anregen oder Verstärken des zellulären Energiestoffwechsels.

Ein weiterer Aspekt betrifft eine Mischung aus oder umfassend Magnesiumcitrat und Calciumcitrat zur Anwendung in einem therapeutischen Verfahren zum Anregen oder Verstärken des Kohlenhydratstoffwechsels, vorzugsweise des Glukose-Stoffwechsels, insbesondere zum Anregen oder Verstärken des zellulären Energiestoffwechsels.

Ein weiterer Aspekt betrifft eine Mischung aus oder umfassend Magnesiumcitrat und Zinkcitrat zur Anwendung in einem therapeutischen Verfahren zum Anregen oder Verstärken des Kohlenhydratstoffwechsels, vorzugsweise des Glukose-Stoffwechsels, insbesondere zum Anregen oder Verstärken des zellulären Energiestoffwechsels.

Ein weiterer Aspekt betrifft eine Mischung aus oder umfassend Magnesiumcitrat, Calciumcitrat und Zinkcitrat zur Anwendung in einem therapeutischen Verfahren zum Anregen oder Verstärken des Kohlenhydratstoffwechsels, vorzugsweise des Glukose-Stoffwechsels, insbesondere zum Anregen oder Verstärken des zellulären Energiestoffwechsels.

Gemäß eines weiteren Aspekts umfasst die Mischung zur Anwendung in einem therapeutischen Verfahren zum Anregen oder Verstärken des Kohlenhydratstoffwechsels, vorzugsweise des Glukose-Stoffwechsels, insbesondere zum Anregen oder Verstärken des zellulären Energiestoffwechsels, zwei, drei, vier, fünf, sechs, sieben, acht, neun, zehn oder mehr verschiedene Citratsalze, die miteinander kombiniert werden, bzw. besteht daraus, wobei ein oder mehrere, vorzugsweise sämtliche, Citratsalz(e) vorzugsweise ausgewählt ist bzw. sind aus der Gruppe bestehend aus Magnesiumcitrat, Calciumcitrat, Zinkcitrat, Natriumcitrat, Kupfercitrat, Eisencitrat, Kaliumcitrat, Kalium-Natrium-Hydrogencitrat, Calcium-Natrium-Hydrogencitrat, di-Natriumhydrogencitrat, Ammoniumcitrat, Ammoniumhydrogencitrat, Molybdäncitrat, Mangancitrat, Lithiumcitrat und Chromcitrat, und wobei mindestens Magnesiumcitrat umfasst ist, und wobei bezüglich der übrigen Citratsalze jegliche Kombinationen der vorstehend genannten Salze vom Umfang der vorliegenden Offenbarung umfasst sind.

Die Mineralstoffe Kalium, Calcium, Magnesium und Natrium gehören zu den sogenannten Mengenelementen, also zu den Mineralien, die der Körper aus der Nahrung aufnimmt und von denen der Körper einen hohen Gehalt benötigt.

Kalium ist unter anderem in der Blutdruckregulation involviert sowie in der Aufrechterhaltung des Membranpotentials, es regelt den Flüssigkeitsgehalt der Zellen und hat zahlreiche weitere Stoffwechselaufgaben wie die Protein- und Glykogenbildung. In den Nerven sorgt Kalium für die Reizweiterleitung, in der Muskulatur ist es an der Steuerung der Kontraktionen beteiligt.

Calcium befindet sich zu 99% im Knochen. Die restlichen 1% spielen als intra- wie extrazellulär gelöste Ca²⁺-Ionen im Blut sowie im Interstitium eine wichtige Rolle bei der extra- und intrazellulären Signalübertragung (z.B. Übertragung von Nervenimpulsen oder Muskelkontraktion), bei der Blutgerinnung und der Aktivierung von Enzymen (M. Peacock, Clin. J. Am. Soc. Nephrol. 2010, 5, 23-30).

Magnesium befindet sich überwiegend im intrazellulären Kompartiment des Körpers und ist essentiell für die Funktion von Enzymen, insbesondere für die Funktion von Enzymen, die Phosphatgruppen übertragen. Magnesium ist erforderlich für den zellulären Energiestoffwechsel und spielt eine wichtige Rolle für Membranstabilisierung, Nervenleitgeschwindigkeit, Ionen-Transport und Calciumkanal-Aktivität (J. R. Weisinger, Lancet 1998, 352, 391-396).

Natrium hat u.a. Bedeutung für das Konzentrationsgefälle in Nervenzellen (zusammen mit Kalium), die Aufnahme und den Transport von Nährstoffen, die Regulation des Wasserhaushaltes und des Säure/Basen-Gleichgewichtes. Natrium fördert zudem die Aufnahme von Nährstoffen wie Glukose und Aminosäuren.

Die Mineralstoffe Chrom, Eisen, Kupfer, Mangan, Molybdän und Zink gehören zu den sogenannten Spurenelementen, also zu den Mineralien von denen der Körper nur eine geringe Menge benötigt, die jedoch essentiell für den Organismus sind und welche er aus der Nahrung aufnimmt.

Chrom ist eines der häufigsten Elemente auf der Erde und kommt als Cr³⁺ in den meisten Nahrungsmitteln vor. Ein Chrom-Mangel entsteht somit kaum. Erst durch Patienten unter parenteraler Ernährung wurde entdeckt, dass ein Chrom-Mangel zu diabetesähnlichen Symptomen führen kann. Zahlreiche darauf folgende Studien kamen zum Ergebnis, dass Chrom eine Rolle im Glucose- und Lipid-Metabolismus spielt (W. T. Cefalu, F. B. Hu, Diabetes Care 2004, 27, 2741-2751).

Eisen wird unter anderem zum Aufbau des Hämoglobins in den roten Blutkörperchen benötigt, zum Sauerstofftransport, der Produktion von stoffwechselanregenden Enzymen und der Energiegewinnung bei Ausdauerleistung.

Kupfer ist gemeinsam mit Eisen für die Aktivierung wichtiger Enzyme notwendig, die Farbbildung in Haut und Haaren, die Produktion roter Blutkörperchen und Aufrechterhaltung des Nervensystems.

Mangan ist im Körper vor allem am Aufbau der Bindegewebe beteiligt, was über die Synthese von Proteoglykanen in Knorpel- und Knochengeweben geschieht. Mangan trägt weiter zur Synthese von Proteinen und Fetten bei, und es wird für die Insulinsynthese und -sekretion sowie für die Bildung von Harnstoff benötigt. Außerdem ist Mangan an der Herstellung von Melanin und Dopamin beteiligt. Mangan aktiviert zudem eine Reihe von Enzymen, die beispielsweise als Antioxidans wirken (Mn-Superoxiddismutase), zur Verwertung von Vitamin B1 beitragen (Phosphatase) und für die Glukoneogenese, d.h. für die Bildung von Glukose, benötigt werden.

Molybdän ist ein Übergangselement und kann durch seine hohe Oxidationszahl gleichzeitig mehrere Elektronen übertragen und ist in der Lage, Sauerstoff auf verschiedene Substrate zu übertragen. Somit ist Molybdän ein wichtiger Co-Faktor für Oxidasen, wie z.B. die Xanthin-Oxidase, welche im Purin-Stoffwechsel involviert ist oder die Sulfit-Oxidase, welche im Stoffwechsel schwefelhaltiger Aminosäuren involviert ist (R. R. Mendel, J. Biol. Chem. 2013, 288 (16), 13165-13172).

Zink hat sowohl katalytische als auch strukturelle Funktionen in Zink-Enzymen und nimmt an Redox-Reaktionen teil, ohne dabei seinen Redox-Zustand zu ändern. Als Bestandteil von Zinkfingerproteinen gibt es dem Protein ein spezielles Gerüst und interagiert somit mit der DNA oder Proteinen (W. Maret, J. Nutr. 2000, 130, 1455-1458). Es hat zudem eine wichtige Funktion bei der Wundheilung, für das Wachstum und die Stärkung des Immunsystems. Zink ist zudem Cofaktor der Carboanhydrase. Diese Enzyme katalysieren die Hydratisierung von Kohlendioxid zu Kohlensäure und umgekehrt. Die Kohlensäure dissoziiert zu Hydrogencarbonat und H⁺. Über diese Reaktion wird der pH-Wert des Blutplasmas und damit der Säure-Basen-Haushalt als auch der pH-Wert der Magensäure reguliert.

Lithium gilt bisher für den Menschen als nicht-essentiell, jedoch ist eine Reihe von therapeutischen Wirkungen, besonders auf die Psyche, nachgewiesen (A. Cipriani, K. Hawton, S. Stockton, J. R. Geddes, BMJ 2013, 346, f3646).

Die Bioverfügbarkeit der Mineralstoff-Kationen hängt stark von deren Gegenionen ab. Es konnte gezeigt werden, dass kationische Mineralstoffe in Verbindung mit organischen Gegenionen besser vom Körper aufgenommen werden können als anorganische Verbindungen. In einer Vergleichsstudie mit verschiedenen Magnesiumverbindungen stellte sich heraus, dass Magnesiumcitrat als einzige Verbindung sowohl nach einmaliger Einnahme als auch nach chronischer Supplementation (60 Tage) zum Anstieg der Magnesium-Plasmakonzentration führte (A. F. Walker, G. Marakis, S. Christie, M. Byng, Magnesium Research 2003, 16, 183-191).

Das Anregen oder Verstärken des Kohlenhydratstoffwechsels und damit des zellulären Energiestoffwechsels mithilfe des/der erfindungsgemäß zu verwendenden Citratsalze(s), wie hierin beschrieben, ist daher besonders vorteilhaft, da die vorangehend beschriebenen Mineralstoff-Kationen essentielle Mengen- bzw. Spurenelemente für den Organismus darstellen und das Citrat-Gegenion die Bioverfügbarkeit der Kationen sicherstellt.

Die Aktivität des Kohlenhydratstoffwechsels kann anhand der Konzentrationswerte bestimmter Analyten beobachtet werden. Dazu zählen unter anderem die Konzentration der Blutglukose, des Insulins, der Glukose in interstitieller Flüssigkeit sowie die Konzentration der Glukose-Metaboliten Lactat und Pyruvat im Blut und in interstitieller Flüssigkeit. Zudem ergeben der Sauerstoffverbrauch und die Kohlendioxidproduktion, unter der Annahme, dass der gesamte verbrauchte Sauerstoff und das gesamte gebildete Kohlendioxid ausschließlich der Oxidation der Hauptnährstoffe entstammen, indirekt Aufschluss über die Aktivität des Energiestoffwechsels. Die Messung und Bestimmung der oben genannten Analyten unter Einsatz gängiger Geräte und unter Verwendung üblichen Probenmaterials, wie arterielles, venöses oder Kapillarblut, Blutserum, Dialysat wie vorangehend definiert oder Ein- und Ausatemluft, ist dem Fachmann auf dem Gebiet, etwa einem Fachmann für klinische Diagnostik, wohlbekannt.

Die Blutglukose (auch "Blutzucker" genannt) bezeichnet die Konzentration von Glukose im Blut. Die Konzentrationswerte des Blutzuckers können in mg/dl oder mmol/l angegeben werden.

Bei Menschen ohne Diabetes liegt der Blutzuckerspiegel im Blut nüchtern, d.h. nach 8 bis 10 Stunden ohne Nahrung, i.d.R. zwischen 55-110 mg/dl (3,1 - 6,1 mmol/l). Nach dem Essen steigt der Blutzucker-Wert gewöhnlich nicht über 140 mg/dl (7,8 mmol/l).

Ein Blutzucker-Nüchternwert zwischen 100 und 125 mg/dl (5,6 bis 6,9 mmol/l) kann auf ein Typ-2-Diabetes-Vorstadium (Prädiabetes) hinweisen. Die Diagnose kann mit weiteren Tests, insbesondere einem oralen Glukosetoleranztest geklärt werden. Ein Diabetes mellitus liegt häufig dann vor, wenn der Blutzucker-Wert nüchtern über 126 mg/dl (7,0 mmol/l) oder zu einem beliebigen Zeitpunkt über 200 mg/dl (11,1 mmol/l) liegt.

Anstatt im Blut kann die Glukosekonzentration auch in interstitieller Flüssigkeit bestimmt werden. Untersuchungen haben gezeigt, dass die gemessene Glukosekonzentration in interstitieller Flüssigkeit nicht direkt mit den im Blut gemessenen Werten korreliert. Im stätionären Zustand erreicht die Glukosekonzentration im Interstitium jedoch einen bestimmten, in der Regel konstanten, Prozentsatz der Blutglukosekonzentration. Dabei beträgt unter stationären Bedingungen die interstitielle Glukosekonzentration zwischen 67 und 106% relativ zur Blutglukose. Bei zeitlichen Änderungen der Glukosekonzentration im Organismus wird dabei ein zeitlicher Versatz von 2 bis 60 Minuten zwischen den Änderungen im Blut und im Interstitium beobachtet, wobei die Glukosekonzentration im Interstitium der Blutglukosekonzentration nacheilt (WO 2001047408 A1).

Die basale Insulinkonzentration im Blut liegt im nüchternen Zustand bei gesunden Menschen i.d.R. zwischen 5 und 30 µU/mL. Zwischen den Mahlzeiten findet in Abhängigkeit von Zusammensetzung und Menge der mit der Nahrung aufgenommenen Kohlenhydrate zwischen 30 und 60 Minuten postprandial eine zeitlich hochvariable, rasche und kurzfristige Stimulation der Insulinkonzentration bis in den Bereich von 50 - 100 µU/ml statt (C. Högermann, MNU 68/1 (15.1.2015) Seiten 1-3, ISSN 0025-5866, Verlag Klaus Seeberger, Neuss).

Die Lactatkonzentration im Plasma beträgt normalerweise zwischen 5,7 und 22 mg/dl (0,63-2,44 mmol/l). Im arteriellen Blut liegen die Werte deutlich niedriger bei i.d.R. < 16 mg/dl (< als 1,8 mmol/l) und im Liquor bei i.d.R. 11 bis 19 mg/dl (1,2-2,1mmol/l). Plasmawerte sind höher als Werte aus Vollblut. Eine Stunde nach einer kohlenhydratreichen Mahlzeit erfolgt ein Antieg auf 120-150% des Nüchternwertes (K. Dörner, Klinische Chemie und Hämatologie (Taschenlehrbuch), 7. Auflage, Thieme Verlag). Ein Ansteigen des Lactats über den Normwert deutet auf eine Herabsetzung des Sauerstoffgehalts bestimmter Gewebsregionen hin, aber auch im Anschluss an viel Bewegung kann der Lactatwert erhöht sein. Steigt der Lactatspiegel im Blut auf > 45 mg/dl (5 mmol/l) wird i.d.R. eine sogenannte Lactat-Azidose angenommen.

Die Pyruvatkonzentration im Plasma beträgt normalerweise zwischen 0,4 und 0,8 mg/dl (45-91 µmol/l). Eine Stunde nach einer kohlenhydratreichen Mahlzeit sind die Werte um bis zu 114 µmol/l (1 mg/dl) höher. Der Quotient Lactat/Pyruvat beträgt normalerweise (10-20)/1 (K. Dörner, Klinische Chemie und Hämatologie (Taschenlehrbuch), 7. Auflage, Thieme Verlag).

Unter Anregen oder Verstärken des Kohlenhydratstoffwechsels im Sinne der vorliegenden Erfindung ist vorzugsweise eine, bevorzugt statistisch signifikante, Veränderung in die Richtung der Erhöhung der Kohlenhydratstoffwechselrate eines oder mehrerer Mess-parameter(s), die mit dem Kohlenhydratstoffwechsel in Verbindung gebracht werden, nach der Intervention (V2) im Vergleich zu vor der Intervention (V1) zu verstehen. Dazu zählen unter anderem die Glukose- und Insulin-Werte in venösem Blut oder Serum (basal und postprandial), die Harnsäure und Magnesiumwerte in venösem Blut oder Serum (basal und postprandial), der Kohlendioxid- und Sauerstoff-Partialdruck im Kapillarblut oder Serum (basal und postprandial), die Bicarbonat-Ionenkonzentration im Kapillarblut oder Serum (basal und postprandial), die postprandiale Thermogenese wie voranstehend definiert, die Kohlenhydratoxidationsrate (basal und postprandial), die Fettoxidationsrate (basal und postprandial), die Durchblutung im Gewebe wie z.B. im Muskel (basal und postprandial), die EtOH-Ratio wie voranstehend definiert (basal und postprandial), die Glukose-, Lactat-, Pyruvat- und Glycerolkonzentration (basal und postprandial) im Dialysat wie voranstehend definiert oder im Blut oder im Urin, der pH-Wert im Dialysat wie voranstehend definiert (basal und postprandial), der Kohlendioxid- und Sauerstoff-Partialdruck (basal und postprandial) im Dialysat wie voranstehend definiert oder im Blut, die Bicarbonat-Ionenkonzentration (basal und postprandial) im Dialysat wie voranstehend definiert oder im Blut, der Ruhe-Nüchtern-Umsatz wie voranstehend definiert, der relative Energieumsatz (basal und postprandial), der respiratorische Quotient wie voranstehend definiert (basal und postprandial). Die Messung und Bestimmung der oben genannten Parameter unter Einsatz gängiger Geräte und unter Verwendung üblichen Probenmaterials, wie arterielles, venöses oder Kapillarblut, Blutserum, Dialysat wie vorangehend definiert oder Ein- und Ausatemluft, und die Auswertung der Parameter in Bezug auf die Kohlenhydratstoffwechselaktivität, ist dem Fachmann auf dem Gebiet, etwa einem Fachmann für klinische Diagnostik, wohlbekannt.

Ein Aspekt der vorliegenden Offenbarung betrifft ein Magnesiumcitrat oder eine Mischung aus zwei, drei oder mehr Citratsalzen zur Anwendung wie hierin beschrieben, wobei das therapeutische Verfahren das Anregen oder Verstärken des oxidativen Glukose-Stoffwechsels, vorzugsweise das Anregen oder Verstärken des oxidativen Glukose-Stoffwechsels im Muskel und/oder das Vorbeugen und/oder Behandeln einer Lactat-Azidose, umfasst.

Im Rahmen der vorliegenden Erfindung konnte überraschenderweise gezeigt werden, dass die Lactat/Pyruvat-Ratio nach einer erfindungsgemäßen Behandlung der Verum-Gruppe mit einem oder mehreren Citratsalz(en) wie hierin beschrieben beeinflusst werden kann. Zur Ermittlung der Lactat/Pyruvat-Ratio wurde mit Hilfe einer in den rechten Oberschenkelmuskel eingebrachten Mikrosonde nach einer Testmahlzeit ein Dialysat gewonnen und das Dialysat anschließend hinsichtlich der enthaltenen Konzentration an Glukose, Lactat und Pyruvat analysiert. Es stellte sich unerwarteterweise heraus, dass die Behandlung der Verum-Gruppe der Probanden mit hierin beschriebenen Citratsalz(en) zu einer signifikanten Erhöhung sowohl der basalen als auch der postprandialen Pyruvat-Konzentration im Dialysat führt und sich dadurch die Lactat/Pyruvat-Ratio stark erniedrigte. Dies weist auf einen deutlich verbesserten oxidativen Glukose-Stoffwechsel unter Behandlung mit dem/den hierin beschriebenen Citratsalz(en) hin.

Durch die Verabreichung des/der erfindungsgemäß zu verwendenden Citratsalze(s) kann daher das Eintreten von Symptomen einer Lactat-Übersäuerung im Organismus verhindert werden bzw. können deren Symptome vermindert werden.

Das Anregen oder Verstärken des oxidativen Kohlenhydratstoffwechsels im Muskel ist dabei besonders vorteilhaft, da es zu einer besonders hohen Energiebereitstellung in den Muskelzellen führt.

Ein weiterer Aspekt betrifft ein Magnesiumcitrat oder eine Mischung aus zwei, drei oder mehr Citratsalzen zur Anwendung wie hierin beschrieben, wobei das therapeutische Verfahren die Behandlung und/oder Vorbeugung einer Stoffwechselerkrankung umfasst, wobei die Stoffwechselerkrankung mindestens eine ausgewählt aus der Gruppe bestehend aus Diabetes mellitus, Adipositas und metabolisches Syndrom umfasst.

Wie voranstehend ausgeführt, führt das Anregen oder Verstärken des Kohlenhydratstoffwechsels mithilfe des/der erfindungsgemäß zu verwendenden Citratsalze(s) zu einer verstärkten aeroben, d.h. vollständigen Verbrennung der Glukose zu Kohlendioxid und Wasser und damit zu einer effektiveren Energiebereitstellung. Dies kann zur Vorbeugung und/oder Behandlung gängiger Stoffwechselerkrankungen wie z.B. Diabetes mellitus, Adipositas und des metabolischen Syndroms genutzt werden.

Bei Diabetes mellitus handelt es sich um eine Stoffwechselerkrankung, bei der die Konzentration des Glukosespiegels im Blut zu hoch ist, da ein absoluter Insulinmangel (Typ 1-Diabetes) oder eine Insulinresistenz, ein Hyperinsulinismus, ein relativer Insulinmangel oder Sekretionsstörungen (Typ 2-Diabetes) vorliegen.

Adipositas, auch als Fettleibigkeit, Obesitas, Obesität oder Fettsucht bekannt, führt zu einem erhöhten Risiko, an einem Typ 2-Diabetes zu erkranken. Durch das Überangebot an Glukose, welche durch über den Energiebedarf hinausgehende Nahrungsmengen aufgenommen wird, und den daraus resultierenden, dauerhaft erhöhten Insulinspiegel sinken die Sensibilität sowie die Anzahl der Insulinrezeptoren. Das ausgeschüttete Insulin reicht nicht mehr aus, um die Glukose abzubauen. Der Körper leidet somit unter einem relativen Insulinmangel und muss vermehrt neues Insulin bilden. Dies führt zu einer Überbeanspruchung der insulinproduzierenden Betazellen der Bauchspeicheldrüse (Pankreas), was wiederum zu einer Erschöpfung des Organs und zu einer Ausbildung eines Typ 2-Diabetes mellitus führt. Zudem haben nicht nur das Überangebot an Glukose, sondern auch die Blutfette einen entscheidenden Einfluss auf das Risiko, einen Typ 2-Diabetes zu entwickeln. Die Menge der im Blut befindlichen freien Fettsäuren hängt mit der Entstehung der Insulinresistenz in Muskulatur und Leber zusammen. Es wird mehr Insulin zur Erhaltung eines normalen Blutglukosespiegels benötigt. Dadurch wirkt sich das Überangebot an Fettsäuren, die durch übermäßigen Verzehr aufgenommen werden, weiterhin ungünstig auf die insulinproduzierenden Betazellen der Bauchspeicheldrüse aus.

Der Begriff metabolisches Syndrom bezeichnet die Kombination von gestörtem Kohlenhydratstoffwechsel, der sich durch eine Insulinresistenz oder erhöhte Glukosekonzentrationen im Blut manifestiert, Bluthochdruck, Dyslipoproteinämie, d.h. Erhöhung der VLDL- bei gleichzeitiger Erniedrigung der HDL-Lipoproteine, und abdomineller Adipositas. Der dem metabolischen Syndrom wahrscheinlich zugrunde liegende Defekt ist eine periphere Insulinresistenz und die damit verbundene chronische Hyperinsulinämie. Das metabolische Syndrom wird als Vorstufe des Typ-2 Diabetes angesehen.

Die vorangehend beschriebenen Stoffwechselerkrankungen haben gemeinsam, dass sie mit einem erhöhten Blutglukosespiegel in Verbindung gebracht werden. Ein Anregen oder Verstärken des Kohlenhydratstoffwechsels mit Hilfe des/der erfindungsgemäß zu verwendenden Citratsalze(s) kann sich daher vorteilhaft auf einen erhöhten Blutglukosespiegel auswirken. Ein geringerer Anstieg der Blutglukose-Werte durch einen angeregten oder verstärkten Kohlenhydratstoffwechsel und einer damit verbundenen geringeren benötigten Insulin-Konzentration nach einer Mahlzeit kann dabei behilflich sein, dem Auftreten der vorgenannten Stoffwechselerkrankungen vorzubeugen bzw. den Schweregrad der Symptome der vorgenannten Stoffwechselerkrankungen zu verringern.

Ein weiterer Aspekt betrifft ein Magnesiumcitrat oder eine Mischung aus zwei, drei oder mehr Citratsalzen zur Anwendung wie hierin beschrieben, wobei das Anregen oder Verstärken des Kohlenhydratstoffwechsels bzw. das Anregen oder Verstärken des oxidativen Glukose-Stoffwechsels zusätzlich durch das/die gewählte(n) Citratsalz(e) die Beeinflussung des Säure-Basen-Haushalts, insbesondere die Beeinflussung des Kohlensäure-Bicarbonat-Systems, bedingt.

Wie vorangehend beschrieben, führt ein erfindungsgemäßes Anregen oder Verstärken des oxidativen Kohlenhydratstoffwechsels zu einem verstärkten Abbau von Glukose über Pyruvat anstelle von Lactat. Lactat ist das Säureanion der Milchsäure und führt zu einer Erniedrigung des pH-Werts im Blut oder in Geweben. Von einer Lactat-Azidose spricht man, wenn der pH-Wert erniedrigt (< 7,36) und gleichzeitig die Lactatkonzentration erhöht ist (> 5mmol/l). Eine Erniedrigung der Lactat/Pyruvat-Ratio durch das Anregen oder Verstärken des oxidativen Kohlenhydratstoffwechsels und einer damit verringerten Bildung von Lactat aus Glukose wirkt sich somit vorteilhaft auf den pH-Wert bzw. Säure-Basen-Haushalt im Körper aus.

Das/die erfindungsgemäß zu verwendende(n) Citratsalz(e) regen dabei nicht nur den Kohlenhydratstoffwechsel an, ihre Verwendung ist zudem besonders vorteilhaft, da Citrate und Hydrogencitrate selbst einen basischen Effekt im Körper ausüben. Sie sind Anionen der Citronensäure, d.h. sie können Säure in Form von Protonen binden und werden anschließend zu den pH-neutralen Stoffwechselprodukten Kohlendioxid und Wasser abgebaut.

Das genaue Wirkprinzip eines Citratsalzes wie vorangehend definiert, soll beispielhaft anhand von Magnesiumcitrat erläutert werden. Aufgenommenes Magnesiumcitrat bleibt von der Magensäure unberührt und gelangt in den oberen Dünndarmabschnitt. Bei einem leicht alkalischen pH-Wert von 8, welcher in diesem Darmabschnitt vorliegt, dissoziiert das Magnesiumcitrat in das Magnesium-Kation, welches gleichzeitig ein wichtiger Mineralstoff ist, und das Anion Citrat und kann dann vom Körper aufgenommen werden. In der Zelle bindet das Citratmolekül aktiv Protonen, wobei sich Citronensäure bildet. Die Citronensäure wird in den Citronensäurezyklus eingeschleust und letztendlich zu Wasser und Kohlendioxid abgebaut. Das Kohlendioxid wird über die Lunge abgeatmet, dass Wasser über den Urin ausgeschieden. Über das Citrat wird somit Säure gebunden und durch die Bildung neutraler Endprodukte aktiv aus dem Körper entfernt.

Die verringerte Bildung von Lactat durch Anregen des oxidativen Glucose-Stoffwechsels zusammen mit der Wirkung des Citrat-Anions auf den pH-Wert verhindert oder verringert eine chronische Übersäuerung des Körpers, welche ansonsten in der Ablagerung überschüssiger Säure im Bindegewebe oder der Herauslösung basischer Mineralstoffe aus den Knochen resultieren würde.

Bindegewebe, welches Säure einlagert, verändert seine physikalisch-chemischen Eigenschaften. In Knochen und Gelenkknorpeln dient das Bindegewebe als kollagenes Stützgewebe. Bei einer Übersäuerung würden sich jedoch an Stelle von Wassermolekülen Protonen an die Gerüstmoleküle (Proteoglykane) des Bindegewebes anlagern und das Bindegewebe würde seine Wasserbindungsfähigkeit verlieren. Dadurch würde das Bindegewebe an Elastizität verlieren, mechanische Belastungen könnten schlechter abgefangen werden und die Entzündungsneigung wäre erhöht. Dies könnte sich durch Muskel- und Gelenkschmerzen bemerkbar machen.

Zum Ausgleich der Säurebelastung bei einer Übersäuerung würden zudem unter anderem Calcium und Magnesium aus den Knochen freigesetzt. Dies würde über einen längeren Zeitraum zu einem Abbau der Knochensubstanz führen.

Eine weitere Ausführungsform betrifft eine Mischung aus drei oder mehr Citratsalzen zur Anwendung wie hierin beschrieben, wobei die Mischung mindestens Magnesiumcitrat, Calciumcitrat und Zinkcitrat umfasst, oder wobei die Mischung aus Magnesiumcitrat, Calciumcitrat und Zinkcitrat besteht.

Eine Mischung umfassend mindestens Magnesiumcitrat, Calciumcitrat und Zinkcitrat oder bestehend aus Magnesiumcitrat, Calciumcitrat und Zinkcitrat zur Anwendung in einem therapeutischen Verfahren zum Anregen oder Verstärken des Kohlenhydratstoffwechsels wirkt sich dabei besonders vorteilhaft aus, da Magnesium, Calcium und Zink, wie vorangehend ausgeführt, wichtige Mengen- bzw. Spurenelemente sind, die der Köper für eine gesunde Funktion in ausreichender Menge benötigt.

Eine weitere Ausführungsform betrifft eine Mischung aus drei oder mehr Citratsalzen zur Anwendung wie hierin beschrieben, wobei, bezogen auf das Gesamtgewicht der Citratsalze, der Anteil an Magnesiumcitrat im Bereich von 2 bis 100 Gew.-% liegt, vorzugsweise im Bereich von bis 2 bis 40 Gew.-%, besonders bevorzugt im Bereich von 9 bis 36 Gew.-%, ganz besonders bevorzugt im Bereich von 15 bis 36 Gew.-%. Gemäß einer alternativen, besonders bevorzugten Ausführungsform liegt der Anteil an Magnesiumcitrat im Bereich von 30 bis 40 Gew.-%.

In einer bevorzugten Ausführungsform beträgt der Anteil an Magnesiumcitrat bezogen auf das Gesamtgewicht der Citratsalze (wenigstens) 2 Gew.-%, 3 Gew.-%, 4 Gew.-%, 5 Gew.-%, 6 Gew.-%, 7 Gew.-%, 8 Gew.-%, 9 Gew.-%, 10 Gew.-%, 11 Gew.-%, 12 Gew.-%, 13 Gew.-%, 14 Gew.-%, 15 Gew.-%, 16 Gew.-%, 17 Gew.-%, 18 Gew.-%, 19 Gew.-%, 20 Gew.-%, 21 Gew.-%, 22 Gew.-%, 23 Gew.-%, 24 Gew.-%, 25 Gew.-%, 26 Gew.-%, 27 Gew.-%, 28 Gew.-%, 29 Gew.-%, 30 Gew.-%, 31 Gew.-%, 32 Gew.-%, 33 Gew.-%, 34 Gew.-%, 35 Gew.-% oder 36 Gew.-%.

Ein weiterer Aspekt der Offenbarung betrifft eine Zusammensetzung zur Anwendung in einem therapeutischen Verfahren, wobei die Zusammensetzung ein Magnesiumcitrat oder eine Mischung aus zwei, drei oder mehr Citratsalzen, umfassend Magnesiumcitrat, als aktiven Wirkstoff enthält, wobei die Mischung aus zwei, drei oder mehr Citratsalzen zusätzlich Calciumcitrat und/oder Zinkcitrat umfasst, wobei das therapeutische Verfahren ein Verfahren zum Anregen oder Verstärken des Kohlenhydratstoffwechsels, vorzugsweise des Glukose-Stoffwechsels, insbesondere zum Anregen oder Verstärken des zellulären Energiestoffwechsels; und/oder zum Anregen oder Verstärken des oxidativen Glukose-Stoffwechsels, vorzugsweise zum Anregen oder Verstärken des oxidativen Glukose-Stoffwechsels im Muskel und/oder zum Vorbeugen und/oder Behandeln einer Lactat-Azidose; und/oder zur Behandlung und/oder Vorbeugung einer Stoffwechselerkrankung, vorzugsweise zur Behandlung und/oder Vorbeugung einer, mehrerer oder sämtlicher Stoffwechselerkrankung(en) ausgewählt aus der Gruppe bestehend aus Diabetes mellitus, Adipositas und metabolischen Syndrom; und/oder zur Beeinflussung des Säure-Basen-Haushalts, insbesondere zur Beeinflussung des Kohlensäure-Bicarbonat-Systems, ist.

Ein aktiver Wirkstoff im Sinne dieser Anmeldung ist vorzugsweise ein Stoff oder ein Stoffgemisch, welcher/welches dazu befähigt ist, die vorangehend definierte Anregung oder Verstärkung des Kohlenhydratstoffwechsels oder des oxidativen Glukose-Stoffwechsels, insbesondere zum Anregen oder Verstärken des zellulären Energiestoffwechsels, vorzugsweise des oxidativen Glukose-Stoffwechsels im Muskel, und/oder das Vorbeugen und/oder Behandeln einer Lactat-Azidose, und/oder die Behandlung und/oder Vorbeugung einer Stoffwechselerkrankung, und/oder die Beeinflussung des Säure-Basen-Haushalts, insbesondere der Beeinflussung des Kohlensäure-Bicarbonat-Systems, in einer hierfür erforderlichen Dosis zu bewirken. Spuren dieser Stoffe bzw. Stoffgemische sind daher regelmäßig nicht als aktiver Wirkstoff aufzufassen. D.h., die hierin beschriebenen erfindungsgemäß zu verwendenden Zusammensetzungen enthalten eine Menge an Citratsalz(en) wie hierin beschrieben, die ausreichend ist, um den bzw. die hierin beschriebenen Effekt(e) zu bewirken.

Laut der deutschen Gesellschaft für Ernährung beträgt die empfohlene Tagesdosis an Magnesium für Jugendliche und junge Erwachsene im Alter von 15 bis 19 Jahren 400 mg (männlich) bzw. 350 mg (weiblich) und für Erwachsene über 25 Jahre 350 mg (männlich) bzw. 300 mg (weiblich).

Der Anteil an Magnesiumcitrat in der erfindungsgemäßen Zusammensetzung wird dabei vorteilhafterweise so gewählt, dass die empfohlene Tagesdosis an Magnesium durch die Verabreichung von Magnesiumcitrat bzw. einer erfindungsgemäß zu verwendenden Mischung aus zwei, drei oder mehr Citratsalzen bzw. einer erfindungsgemäß zu verwendenden Zusammensetzung abgedeckt wird. Hierbei kann die Tagesdosis durch Verabreichung der erfindungsgemäß zu verwendenden Stoffe, Mischungen oder Zusammensetzung einmal, zweimal, dreimal oder mehr als dreimal täglich erreicht werden. Bevorzugt erfolgt die Verabreichung einmal, zweimal oder dreimal täglich.

In einer bevorzugten Ausführungsform beträgt die Tagesdosis an Magnesiumcitrat in einem hierin beschriebenen Verfahren 15 bis 1000 mg.

Eine Ausführungsform betrifft eine Zusammensetzung zur Anwendung in einem therapeutischen Verfahren wie hierin beschrieben, wobei die Zusammensetzung zudem unabhängig voneinander einen oder mehrere weitere Bestandteile ausgewählt aus der Gruppe bestehend aus Süßungsmittel, vorzugsweise Laktose, Saccharose oder Sorbit; Säureregulatoren, vorzugsweise Kaliumtartrat; Trennmittel, vorzugsweise Salze, insbesondere Magnesium- oder Calciumsalze, von Speisefettsäuren, vernetzte Natriumcarboxymethylcellulose oder Siliciumdioxid; Säuerungsmittel, vorzugsweise Zitronensäure; Aromen, vorzugsweise natürliche Aromen; Füllstoffe, vorzugsweise Hydroxypropylcellulose; Magnesiumoxid; weitere Citratsalze, vorzugsweise Natriumcitrat, Kaliumcitrat, Kalium-Natrium-Hydrogencitrat, Calcium-Natrium-Hydrogencitrat, Kupfercitrat und/oder Eisencitrat; Calciumcarbonat, Calciumlactat, Magnesiumcarbonat und/oder Natriumhydrogencarbonat; Vitamine, vorzugsweise Riboflavin, Cholecalciferol (Vitamin D) und/oder Ascorbinsäure; L-Carnitin; Maltodextrin; Molybdän, vorzugsweise Natriummolybdat; Chrom, vorzugsweise Chromchlorid; und Selen, vorzugsweise Natriumselenit, Natriumselenat oder Selenhefe; umfasst.

Weitere pharmazeutisch verträgliche Zusatzstoffe, welche von der Zusammensetzung gemäß der vorliegenden Offenbarung umfasst sein können, sind dem Fachmann auf dem Gebiet wohlbekannt.

Eine weitere Ausführungsform betrifft eine Zusammensetzung zur Anwendung in einem therapeutischen Verfahren wie hierin beschrieben, wobei die Gesamtmenge an Citratsalz(en) in der Zusammensetzung, bezogen auf das Gesamtgewicht der Zusammensetzung, im Bereich von 4 bis 70 Gew.-% liegt, vorzugsweise im Bereich von 8 bis 67 Gew.-%, besonders bevorzugt im Bereich von 19 bis 44 Gew.-%, ganz besonders bevorzugt im Bereich von 25 bis 35 Gew.-%, insbesondere im Bereich von 30 bis 35 Gew.-%.

In einer Ausführungsform beträgt die Gesamtmenge an Citratsalz(en) in der Zusammensetzung, bezogen auf das Gesamtgewicht der Zusammensetzung, (wenigstens) 4 Gew.-%, 5 Gew.-%, 6 Gew.-%, 7 Gew.-%, 8 Gew.-%, 9 Gew.-%, 10 Gew.-%, 11 Gew.-%, 12 Gew.-%, 13 Gew.-%, 14 Gew.-%, 15 Gew.-%, 16 Gew.-%, 17 Gew.-%, 18 Gew.-%, 19 Gew.-%, 20 Gew.-%, 21 Gew.-%, 22 Gew.-%, 23 Gew.-%, 24 Gew.-%, 25 Gew.-%, 26 Gew.-%, 27 Gew.-%, 28 Gew.-%, 29 Gew.-%, 30 Gew.-%, 31 Gew.-%, 32 Gew.-%, 33 Gew.-%, 34 Gew.-%, 35 Gew.-%, 36 Gew.-%, 37 Gew.-%, 38 Gew.-%, 39 Gew.-%, 40 Gew.-%, 41 Gew.-%, 42 Gew.-%, 43 Gew.-%, 44 Gew.-%, 45 Gew.-%, 46 Gew.-%, 47 Gew.-%, 48 Gew.-%, 49 Gew.-%, 50 Gew.-%, 51 Gew.-%, 52 Gew.-%, 53 Gew.-%, 54 Gew.-%, 55 Gew.-%, 56 Gew.-%, 57 Gew.-%, 58 Gew.-%, 59 Gew.-%, 60 Gew.-%, 61 Gew.-%, 62 Gew.-%, 63 Gew.-%, 64 Gew.-% oder 65 Gew.-%.

Eine weitere Ausführungsform betrifft eine Zusammensetzung zur Anwendung in einem therapeutischen Verfahren wie hierin beschrieben, wobei die Zusammensetzung ausgewählt ist aus der Gruppe bestehend aus Granulaten, vorzugsweise zur Direkteinnahme, zum Auflösen oder zum Einrühren in Speisen, Tabletten, Kapseln und Dragees, vorzugsweise Tabletten, Kapseln und Dragees zum Schlucken, Lutschen oder Kauen, Pulver, vorzugsweise zur Direkteinnahme und wasserlösliche Pulver zum Trinken, Trinkampullen und Mikroperlen, vorzugsweise zur direkten Einnahme ohne Wasser.

Weitere Darreichungsformen einer peroralen oder auch topischen Zusammensetzung, die gemäß der vorliegenden Offenbarung umfasst sein können, und die entsprechenden Techniken der Arzneistoff-Formulierung sind dem Fachmann auf dem Gebiet der pharmazeutischen Technologie bekannt und können unter Heranziehung von allgemeinem Fachwissen auf dem Gebiet ohne Weiteres bereitgestellt werden.

Eine weitere Ausführungsform betrifft eine Zusammensetzung zur Anwendung in einem therapeutischen Verfahren wie hierin beschrieben, wobei die Zusammensetzung frei von einem, mehreren oder sämtlichen der Bestandteile ausgewählt aus der Gruppe bestehend aus Laktose, Iod, Gluten, Aromastoffe und Süßungsmitteln ist, vorzugsweise frei ist von Laktose, Iod und Gluten.

Eine weitere Ausführungsform betrifft eine Zusammensetzung zur Anwendung in einem therapeutischen Verfahren wie hierin beschrieben, wobei das therapeutische Verfahren die kontrollierte Freisetzung der aktiven Wirkstoffe der Zusammensetzung verabreicht als Depotform umfasst.

Eine Depotform eines Arzneimittels sorgt für eine verzögerte, möglichst gleichmäßige Wirkstoffabgabe aus einem Wirkstoffvorrat unter Vermeidung von Plasmakonzentrationsspitzen über eine bestimmte Zeit. Dadurch können die totalen Wirkstoffmengen oft reduziert werden, Arzneimittelnebenwirkungen verringert werden und eine mehrmalige Dosierung pro Tag durch eine einmalige Gabe ersetzt werden. Diese Vorteile verbessern die Compliance der Patienten.

Die Anforderungen an eine ideale Depotarzneiform lassen sich also im Wesentlichen folgendermaßen zusammenfassen:
- Von der Zubereitung wird nach der Applikation ein rasches Erreichen der therapeutisch optimalen Blutspiegelwerte verlangt.
- Es ist ein konstantes Blutspiegelniveau zu sichern.
- Über die gewünschte Zeitdauer muss eine gleichmäßige biologische Wirkung erhalten bleiben.
- Durch die Vermeidung von Konzentrationsspitzen, d.h. Verhinderung des Vordringens der Wirkstoffkonzentration in den toxischen Bereich, sind Intensität und Häufigkeit unerwünschter Nebenwirkungen zu reduzieren.

Nach der umfassenden Definition der FDA (Food and Drug Administration) werden unter "controlled realease products" Formulierungen verstanden, die bestimmt sind, den aktiven Bestandteil in einer Weise freizusetzen, die sich signifikant von den entsprechenden Zubereitungen mit sofortiger Freisetzung unterscheiden. Diese Definition schließt alle Typen von Modified-release-(Depot-)Arzneiformen sowie solche mit zeitlich fixierter Freisetzung ein. Auch der Begriff Arzneiformen mit modifizierter Freisetzung" fasst alle Arzneiformen zusammen, die beabsichtigt eine andersartige Freisetzung besitzen als eine normale, schnelle Freisetzung.

Der Begriff Depot-Arzneiform wird oft als übergeordnete Bezeichnung für Arzneiformen mit verlängerter Wirkung verwendet bzw. als Synonym für Modified-release-Arzneiformen.

Es sind vier Typen von Depotarzneiformen zu unterscheiden:
- Sustained-release-Typ (Synonyme: gleichmäßig hinhaltende Wirkstofffreigabe, sustained action): Aus einer Arzneiform wird der Wirkstoff durch eine Initialdosis dem Körper in einer Konzentration zugänglich gemacht, die die gewünschte pharmakodynamische Wirkung ergibt und die eine Erhaltung dieser pharmakologisch optimalen Konzentration für eine gewisse Zeit über die Wirkungszeit einer Einzeldosis hinaus garantiert.
- Prolonged-release-Typ (Synonyme: verlängerte (protrahierte) Wirkstofffreigabe, prolonged action): Aus einer Arzneiform wird der Wirkstoff durch eine Initialdosis dem Körper in einer Menge zugänglich gemacht, die genügend, aber nicht unerwünscht hoch ist und den gewünschten phamakodynamischen Effekt ausübt. Zudem soll diese Arzneiform den Wirkstoff kontinuierlich so freigeben, dass eine messbare Wirkungsverlängerung gegenüber einer normalen Einzeldosis resultiert.
- Repeat-release-Typ (Synonyme: gestaffelte Wirkstofffreigabe, repeat action): Aus der Arzneiform wird vom Wirkstoff zunächst eine Initialdosis und nach einiger Zeit eine weitere Einzeldosis stoßweise freigesetzt. Eventuell können weitere Dosen zur gegebenen Zeit folgen.
- Delayed-release-Typ (Synonym: verzögerte Wirkstofffreigabe): Aus der Arzneiform wird der Wirkstoff erst längere Zeit nach der Applikation freigesetzt.
   (R. Voigt, Pharmazeutische Technologie, 10. Auflage, Deutscher Apotheker Verband Stuttgart).

Die gängigen Techniken der Arzneistoff-Formulierung von Wirkstoffen zu Depotformen, der Einsatz dazu benötigter Gerätschaften und die Verwendung üblicher Substanzen sind dem Fachmann auf dem Gebiet der pharmazeutischen Technologie bekannt und können unter Heranziehung von allgemeinem Fachwissen auf dem Gebiet ohne Weiteres bereitgestellt werden.

Ein weiterer Aspekt der Offenbarung betrifft die nicht-therapeutische Verwendung eines Citratsalzes, nämlich eines Magnesiumcitrates, oder einer Mischung aus zwei, drei oder mehr Citratsalzen wie hierin beschrieben oder einer Zusammensetzung wie hierin beschrieben, zum Erreichen einer oder mehrerer nichttherapeutischer/therapeutischen Wirkung(en) unabhängig voneinander ausgewählt aus der Gruppe bestehend aus: Anregen oder Verstärken des Kohlenhydratstoffwechsels, vorzugsweise des Glukose-Stoffwechsels, insbesondere zum Anregen oder Verstärken des zellulären Energiestoffwechsels, bevorzugt Anregen oder Verstärken des oxidativen Glukose-Stoffwechsels, vorzugsweise Anregen oder Verstärken des oxidativen Glukose-Stoffwechsels im Muskel; Vorbeugung und/oder Verringerung eines Muskelkaters; Gewichtsreduktion; Konzentrationssteigerung; Verdauungsförderung; Verbesserung der körperlichen und geistigen Leistungsfähigkeit; Beeinflussung des Säure-Basen-Haushalts, insbesondere Beeinflussung des Kohlensäure-Bicarbonat-Systems; Vorbeugung und/oder Verringerung von Cellulite, Vorbeugung und/oder Verringerung von Rissen im Bindegewebe, insbesondere von Schwangerschaftsstreifen.

Die Verwendung von Magnesiumcitrat oder einer Mischung aus zwei, drei oder mehr Citratsalzen wie hierin beschrieben oder einer Zusammensetzung wie hierin beschrieben zum Anregen oder Verstärken des Kohlenhydratstoffwechsels, vorzugsweise des Glukose-Stoffwechsels, insbesondere zum Anregen oder Verstärken des zellulären Energiestoffwechsels, kann zur Verbesserung der Symptome einer Reihe von nicht-pathologischen Zuständen herangezogen werden.

Das Anregen oder Verstärken des Glukose-Stoffwechsels, insbesondere des oxidativen Glukose-Stoffwechsels führt, wie vorangehend ausführlich beschrieben, zu einem verminderten Abbau der Glukose über den anaeroben Glukose-Stoffwechselweg und damit zu einer verringerten Bildung von Lactat. Die Vermeidung der "Stoffwechselsackgasse" Lactat und die vollständige Verwertung der Glukose durch den Abbau zu Pyruvat und der anschließenden Verstoffwechslung unter aeroben Bedingungen zu Acetyl-CoA, welches über den Citratzyklus weiter zu CO₂ und Wasser oxidiert wird, ist unter dem Gesichtspunkt der Energiegewinnung des Organismus besonders vorteilhaft. Während der aeroben Oxidation von einem Mol Glucose zu CO₂ wird dabei circa 20 Mal mehr Energie in Form des Energieäquivalents ATP (Adenosintriphosphat) gewonnen als beim anaeroben Abbau zu Lactat, d.h. der zelluläre Energiestoffwechsel wird mit dem/den erfindungsgemäß zu verwendenden Citratsalz(en) angeregt oder verstärkt.

In einer Ausführungsform führt das Anregen oder Verstärken des Kohlenhydratstoffwechsels, insbesondere des oxidativen Glukoseabbaus, mit dem/den erfindungsgemäß zu verwendenden Citratsalz(en) zu einer verbesserten Verwertung der aus der Nahrung aufgenommenen Kohlenhydrate und damit zu einer geringeren Lactatkonzentration und zu einer erhöhten Energiebereitstellung, sowohl systemisch als auch lokal, wie zum Beispiel im Muskel. Dies führt wiederum zu einer generellen Verbesserung der körperlichen und geistigen Leistungsfähigkeit und einer Steigerung der Konzentration, insbesondere in Zeiten erhöhten Energiebedarfs. Müdigkeit und Erschöpfung werden ebenfalls reduziert.

Eine Ausführungsform betrifft die Verhinderung eines nicht-pathologischen Lactatüberschusses im Muskel durch das Anregen oder Verstärken des Kohlenhydratstoffwechsels mit dem/den erfindungsgemäß zu verwendenden Citratsalz(en).

Die gleichzeitige Zuführung essentieller und nicht-essentieller Mengen- und Spurenelemente, welche in dem/den erfindungsgemäß zu verwendenden Citratsalz(en) enthalten sind, führt weiterhin, wie vorangehend beschrieben, zu einer Verbesserung verschiedenster Körperfunktionen, wie zum Beispiel des zellulären Energiestoffwechsels, zu einer Verbesserung des allgemeinen Wohlbefindens und einer Reduktion von Müdigkeit und Erschöpfung.

In einer weiteren Ausführungsform kann das Anregen oder Verstärken des Kohlenhydratstoffwechsels mithilfe des/der erfindungsgemäß zu verwendenden Citratsalze(s) zudem mit einer Förderung der Verdauung und einer unterstützenden Wirkung bei der Gewichtsreduktion in Verbindung gebracht werden. Unter Gewichtsreduktion ist in diesem Zusammenhang eine Reduktion an Köpergewicht von wenigen Kilogramm aus kosmetischen, nicht-therapeutischen Gründen zu verstehen.

Mit der Nahrung aufgenommene Kohlenhydrate werden zunächst im Darm zu Monosacchariden wie zum Beispiel Glukose abgebaut, anschließend werden die in der Verdauung gewonnenen Monosaccharide über das Blut in die Zellen transportiert. Durch einen mit dem/den erfindungsgemäß zu verwendenden Citratsalz(en) angeregten oder verstärkten Kohlenhydratstoffwechsel bzw. zellulären Energiestoffwechsel wird die aufgenommene Glukose direkt durch Energiegewinnung verwertet und nicht im Organismus eingelagert, was sich vorteilhaft auf eine angestrebte Gewichtsreduktion auswirkt.

Wie vorangehend ausgeführt, kann mit dem/den erfindungsgemäß zu verwendenden Citratsalz(en) durch eine Verringerung der Lactatproduktion durch das Anregen des oxidativen Kohlenhydratstoffwechsels und durch den Einfluss des Citratanions auf den pH-Wert im Organismus eine Übersäuerung des Körpers verhindert bzw. vermindert werden.

Ein Aspekt betrifft die Verwendung des/der hierin beschriebenen Citratsalz(e) zur Beeinflussung des Säure-Basen-Haushalts, insbesondere zur Beeinflussung des Kohlensäure-Bicarbonat-Systems, zur Verhinderung und/oder Verbesserung von nicht-pathologischen Bindegewebszuständen wie zum Beispiel der Cellulite (auch Orangenhaut genannt) oder von Rissen im Bindegewebe, vorallem während einer Schwangerschaft (sogenannte Schwangerschaftsstreifen). Wie vorangehend ausgeführt, führt eine Übersäuerung des Körpers zu einer Anlagerung von Protonen anstelle von Wassermolekülen an die Proteoglykane des Bindegewebes, was zu einer Verringerung der Elastizität des Bindegewebes führt. Dieser Zustand kann durch den regulierenden Einfluss des/der erfindungsgemäß zu verwendenden Citratsalze(s) auf den Säure-Basen-Haushalt des Körpers verhindert bzw. verbessert werden.

Das im Rahmen einer hierin beschriebenen Ausführungsform der vorliegenden Erfindung Gesagte (z.B. betreffend (bevorzugte) Bestandteile und Mengen der hierin beschriebenen Citratsalze oder deren Anwendung) gilt selbstverständlich jeweils auch für andere hierin beschriebene Ausführungsformen. Dementsprechend sind die hierin beschriebenen Ausführungsformen beliebig - soweit für den Fachmann sinnvoll - miteinander kombinierbar.

Im Folgenden wird die vorliegende Erfindung anhand ausgewählter Beispiele beschrieben, ohne den anspruchsgemäßen Gegenstand darauf zu beschränken.

### Beispiele:

### I. Zusammensetzungen:

**1. Mikroperlen**

| **Stoff** | **Menge [mg]** |
|---|---|
| Sorbit | 1078,16 |
| Calciumcitrat | 569,26 |
| Kaliumtartrat | 481,23 |
| Magnesiumcitrat | 317,65 |
| Magnesiumoxid | 286,90 |
| E 470a | 28,00 |
| Zinkcitrat | 8,01 |
| Zitrusfruchtextrakt auf Trägerstoff Maltodextrin und Antioxidantien | 3,00 |
| Natriummolybdat | 0,06 |
| Chromchlorid | 0,10 |
| Natriumselenit | 0,03 |

| | |
|---|---|
| Herstellung: Es wird eine Mischung der Stoffe hergestellt und anschließend granuliert. | |

**2. Granulat**

| **Stoff** | **Menge [mg]** |
|---|---|
| Lactose | 25388,49 |
| Calciumcitrat | 2609,70 |
| Natriumcitrat | 1598,90 |
| Magnesiumcitrat | 966,73 |
| Kaliumcitrat | 975,80 |
| Maltodextrin | 408,70 |
| Zinkcitrat | 16,18 |
| Eisencitrat | 23,81 |
| Kupfercitrat | 8,33 |
| Chromchlorid | 0,21 |
| Natriummolybdat | 0,13 |
| Natriumselenit | 0,07 |

| | |
|---|---|
| Herstellung: Wässrige Spurenelementemischung wird auf Trägersubstanz aufgesprüht und granuliert. Aus diesem Granulat wird die Endmischung hergestellt und granuliert. | |

**3. Trinkpulver**

| **Stoff** | **Menge [mg]** |
|---|---|
| Inulin | 2599,87 |
| Natriumcitrat | 1172,71 |
| Kaliumcitrat | 829,65 |
| Calciumlactat | 769,23 |
| Magnesiumcitrat | 618,81 |
| Zinkcitrat | 8,01 |
| Kupfercitrat | 1,42 |
| Chromchlorid | 0,15 |
| Natriummolybdat | 0,10 |
| Natriumselenit | 0,05 |

| | |
|---|---|
| Herstellung: Wässrige Spurenelementemischung wird auf Trägersubstanz aufgesprüht und granuliert. Aus diesem Granulat wird die Endmischung hergestellt. | |

**4. Kapseln**

| **Stoff** | **Menge [mg]** |
|---|---|
| Natriumbicarbonat | 750,00 |
| Hydroxypropylmethylcellulose | 95,00 |
| Cellulose | 12,30 |
| Calciumsalze von Speisefettsäuren E 470a | 3,85 |
| Siliciumdioxid | 3,85 |
| Magnesiumcitrat | 638,30 |
| Gelatine | 100,00 |
| Emulgator: Mono- und Diglyceride von Speisefettsäuren | 80,00 |
| Magnesiumsalze von Speisefettsäuren E 470b | 15,00 |

| | |
|---|---|
| Herstellung: Herstellung von zwei getrennten Kapselmischungen, mit Magnesiumcitrat bzw. Natriumbicarbonat als Hauptsubstanz. Abfüllung in Gelatine- bzw. HPMC-Kapseln. | |

### II. Studienergebnisse:

### 1. Durchführung

Unter Verwendung der in Beispiel 1 definierten Zusammensetzung als Verum, wie vorangehend definiert, wurde eine randomisierte, zweiarmige, doppelblinde Interventionsstudie, wie vorangehend definiert, an 40 Probanden durchgeführt, um den Einfluss der erfindungsgemäß zu verwendenden Zusammensetzung auf den Kohlenhydratstoffwechsel sowie den zellulären Energiestoffwechsel zu untersuchen.

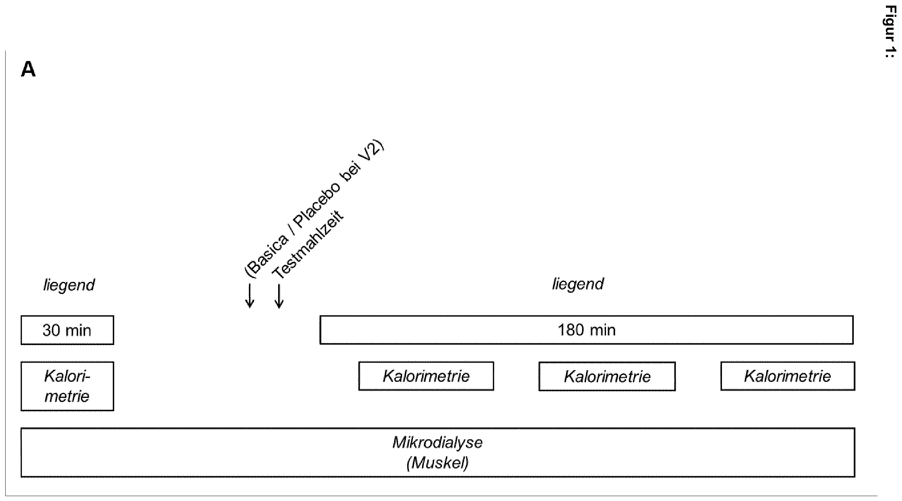

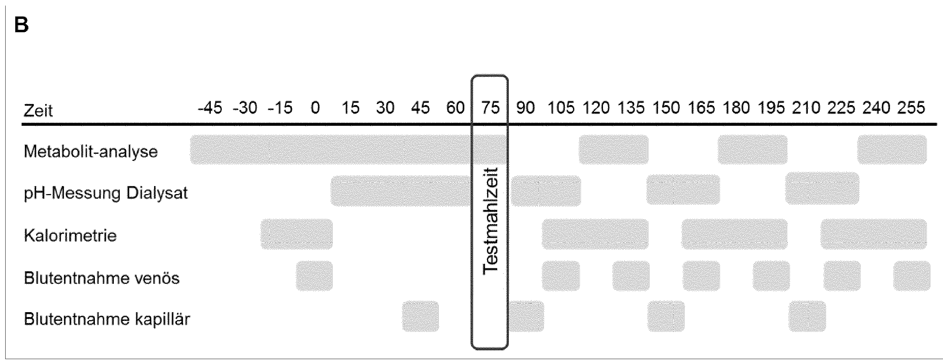

Die Probanden nahmen an zwei vom Studienprotokoll her identischen Untersuchungstagen (V1 vor Intervention bzw. V2 nach Intervention), mit einem Abstand von vier Wochen, teil. Figur 1 zeigt dazu eine detaillierte Übersicht über den Ablauf eines Untersuchungstages.

Nach Abschluss des ersten Untersuchungstages (V1) erhielten die Probanden entweder das Verum, wie vorangehend definiert, (Zusammensetzung wie in Beispiel 1 definiert) oder Placebo, wie vorangehend definiert, das sie über die folgenden vier Wochen morgens und abends zu sich nahmen. Das Aussehen und die Verpackung des Verums sowie des Placebos war dabei identisch.

Die Probanden erschienen am jeweiligen Untersuchungstag (V1 bzw. V2) um 8 Uhr morgens nüchtern und waren instruiert, am Vorabend die letzte Mahlzeit bis spätestens 20 Uhr einzunehmen. Zusätzlich waren sie gebeten worden, innerhalb von 24 h vor den Untersuchungen auf koffein- und alkoholhaltige Getränke und Lebensmittel zu verzichten.

Nach einer 30-minütigen Ruhephase wurde eine Mikrodialyse-Sonde, wie vorangehend definiert, in den rechten Oberschenkel-Muskel der Probanden platziert. Diese wurde anschließend für zunächst 30 min perfundiert, um das inter-/extrazelluläre Gleichgewicht des Gewebes wiederherzustellen. Danach wurde der Ruhe-Nüchtern-Umsatz, wie vorangehend definiert, über 30 min mittels indirekter Kalorimetrie mit Hilfe einer über dem Kopf befindlichen Haube gemessen. Anschließend verzehrten die Probanden eine proteinreiche Testmahlzeit. Der postprandiale Energieumsatz wurde über 180 min gemessen (3 Intervalle à 45 min mit jeweils 15 min Pause). Vor und nach der Testmahlzeit wurde zudem in festen Zeitabständen Kapillarblut aus dem Ohrläppchen der Probanden gewonnen. Zusätzlich wurde ebenfalls in festen Zeitabständen venöses Blut vor und nach der Testmahlzeit abgenommen. Die Mikrodialyse lief kontinuierlich bis zum Ende der Untersuchung (siehe Figur 1).

### 2. Ergebnisse

### 2.1 Blutparameter

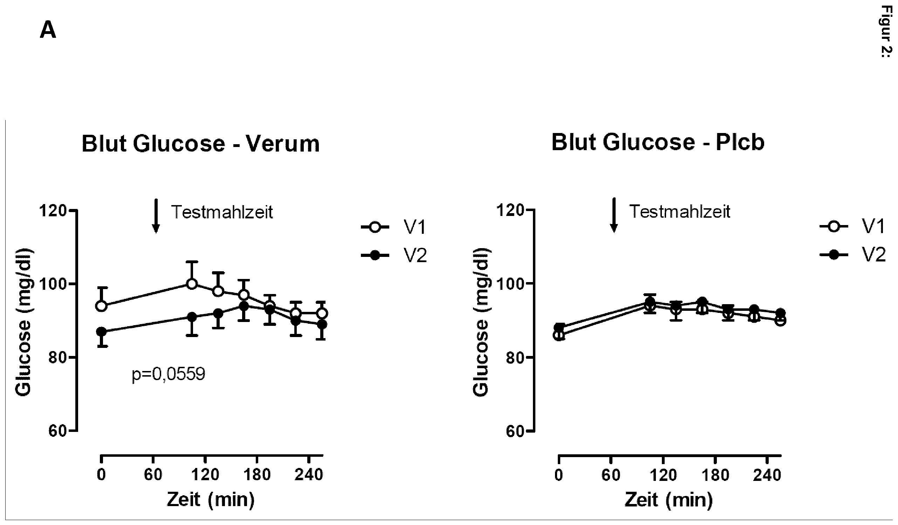

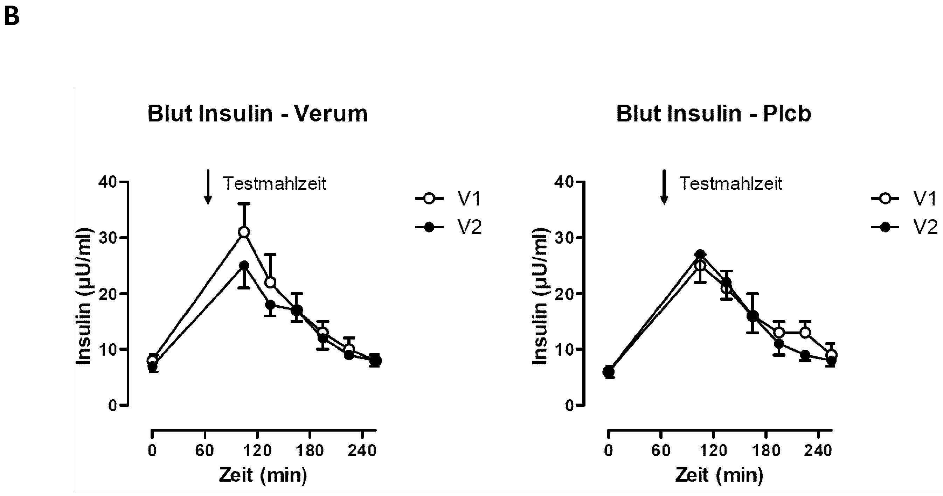

In Figur 2 sind die basalen und postprandialen Blut-Glucose-Konzentrationen vor (V1) und nach (V2) Intervention (A) und die basalen und postprandialen Insulin (B)-Konzentrationen vor (V1) und nach (V2) Intervention (B) dargestellt.

Bei den Probanden beider Gruppen lagen die Werte der Blutglukose und der Insulinkonzentration im Normbereich von nüchternen Gesunden wie vorangehend definiert, so dass eine diabetische Stoffwechsellage ausgeschlossen werden konnte. Nach der Testmahlzeit kam es zu einem leichten Anstieg der Blutglucose - die Werte überschritten jedoch zu keinem Zeitpunkt den Normbereich für Nüchternglucose. Wie in Figur 2 ersichtlich, waren nach einer vierwöchigen Supplementation mit der Zusammensetzung wie in Beispiel 1 definiert (Verum) die basalen und postprandialen Gukose-Werte erniedrigt. Der postprandiale Anstieg von Insulin im Serum unter Supplementation von Verum war ebenfalls niedriger als unter Placebo.

Figur 3 zeigt die basale Serum-Magnesium-Konzentrationen vor (V1) und nach (V2) Intervention.

Die Serum-Magnesium-Konzentrationen waren in der Verum- sowie in der Placebo Gruppe im Normbereich von 0,75 bis 0,95 mmol/L, erhöhten sich jedoch trotzdem signifikant in der Verum-Gruppe nach vierwöchiger Supplementation mit der Zusammensetzung wie in Beispiel 1 definiert.

Figur 4 zeigt die basalen und postprandialen Blut-pO₂-Werte vor (V1) und nach (V2) Intervention.

Die arterielle Sauerstoff-Partialdruck (pO₂) liegt normalerweise zwischen 70-100 mmHg und sinkt mit zunehmendem Alter. In der vorliegenden Studie wurde jedoch Kapillarblut verwendet. In der Literatur gibt es Hinweise, dass die Werte aus dem Kapillarblut nicht dem arteriellen Blut entsprechen. Der Nüchtern-pO₂ entsprach trotzdem dem Normwert (83 ± 5 mmHg) in beiden Gruppen. Nach der Testmahlzeit sank der pO₂ unter Placebo gleichermaßen in V1 und V2, unter Verum in V1 und noch stärker unter V2. Dies deutet darauf hin, dass die Stoffwechselaktivität und damit der Sauerstoffverbrauch unter Verum signifikant stärker war als zuvor.

### 2.2 Energieumsatz

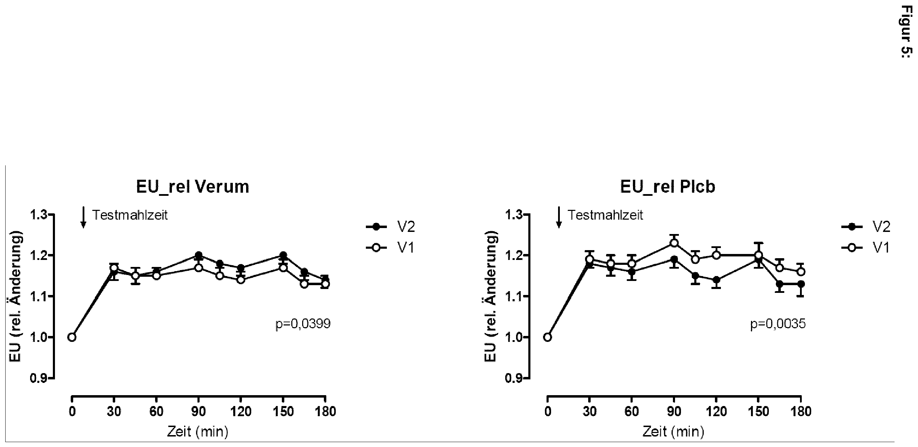

Figur 5 zeigt die relativen Änderungen im Energieumsatz (EU) vor (V1) und nach (V2) Intervention.

Der RNU, wie vorangehend definiert, lag in V1 der Verum-Gruppe bei 3,9 kJ/min und in V1 der Placebo-Gruppe bei 3,8 kJ/min. Nach Einnahme der proteinreichen Testmahlzeit kam es erwartungsgemäß zu einem deutlichen Anstieg im Energieumsatz wie vorangehend definiert. Dieser Prozess wird als postprandiale Thermogenese bezeichnet (wie vorangehend definiert). Die prozentuale postprandiale Umsatzsteigerung lag bei 15% bis 23%, was durchaus dem thermogenen Potential der Proteine in der Testmahlzeit entspricht.

Der relative Anstieg im postprandialen Energieumsatz fiel nach Verum-Gabe eher höher, nach Placebo eher niedriger aus. Ein Grund für den geringeren postprandialen Anstieg im Energieumsatz unter Placebo könnte sein, dass die älteren, in der Regel studienunerfahrenen Probanden in V1 etwas aufgeregt waren und daher die postprandiale Thermogenese zusätzlich noch durch eine stress-bedingte Erhöhung des Energieumsatzes überlagert worden war. In V2 waren die Probanden dann möglicherweise weniger aufgeregt, so dass der postprandiale Anstieg insgesamt geringer ausfiel. Stellt man die gleichen Überlegungen für die Verum-Gruppe an, dann resultiert die höhere Thermogenese aus einem Effekt des Verums und dieser war möglicherweise größer als er sich darstellt, insbesondere wenn man direkt die postprandiale Thermogenese in V2 zwischen Verum und Placebo vergleicht.

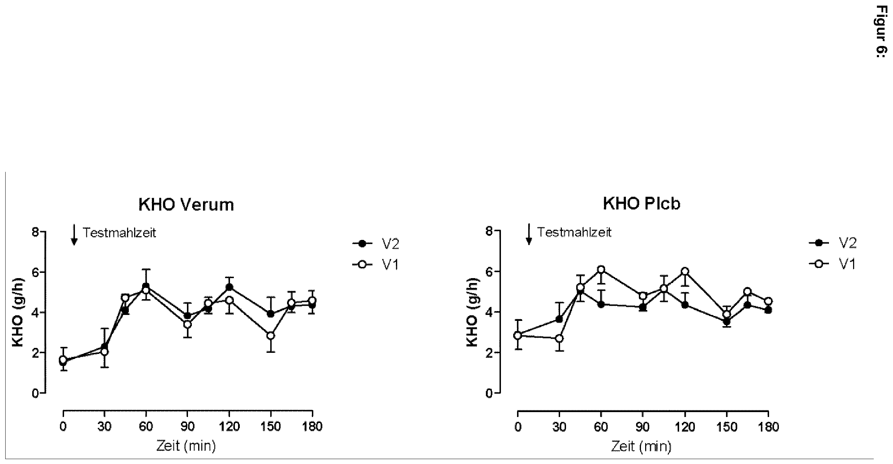

Figur 6 zeigt die basale und postprandiale Kohlenhydratoxidationsrate (KHO) vor (V1) und nach (V2) Intervention (A).

Bei der Testmahlzeit kamen 72% der Energie aus dem Protein und 24% aus den Kohlenhydraten. Interessanterweise wurde der Anstieg im postprandialen Energieumsatz hauptsächlich durch einen Anstieg in der Kohlenhydratoxidation gespeist. Dieser Effekt war unter Verum eher verstärkt, unter Placebo eher verringert.

### 2.3 Mikrodialyse

In der vorliegenden Studie wurde die Mikrodialyse-Technik, wie vorangehend definiert, verwendet, um den Effekt des Verums auf den Stoffwechsel speziell im Muskel zu untersuchen. Das gewonnene Dialysat, wie vorangehend definiert, wurde im Folgenden analysiert. Bei dieser "tissue monitoring technique" ist es immer auch notwendig, die Veränderungen in der lokalen Durchblutung zu erfassen, da die lokale Durchblutung nicht nur die Substratversorgung sondern auch die Produktentsorgung und damit die lokalen interstitiellen Metabolit-Konzentrationen entscheidend beeinflusst. In der vorliegenden Studie wurde die Ethanol-Dilutionstechnik, wie vorangehend bei der Definiton der EtOH-Ratio beschrieben, genutzt, um die relativen Änderungen in der Durchblutung semiquantitativ im Gewebe abschätzen zu können. Da die Probanden während der Untersuchungen im Bett lagen und ihre Position nicht wesentlich änderten, war keine Änderung der Durchblutung infolge von Muskelarbeit zu erwarten. In der postprandialen Phase sind die Verdauungsorgane stärker durchblutet als die Peripherie, beispielsweise die Muskulatur. Darin liegt die Ursache für den kurzzeitigen leichten postprandialen Anstieg der EtOH-Ratio im Muskel, was auf eine leicht reduzierte Durchblutung im Muskel zurückgeführt werden kann.

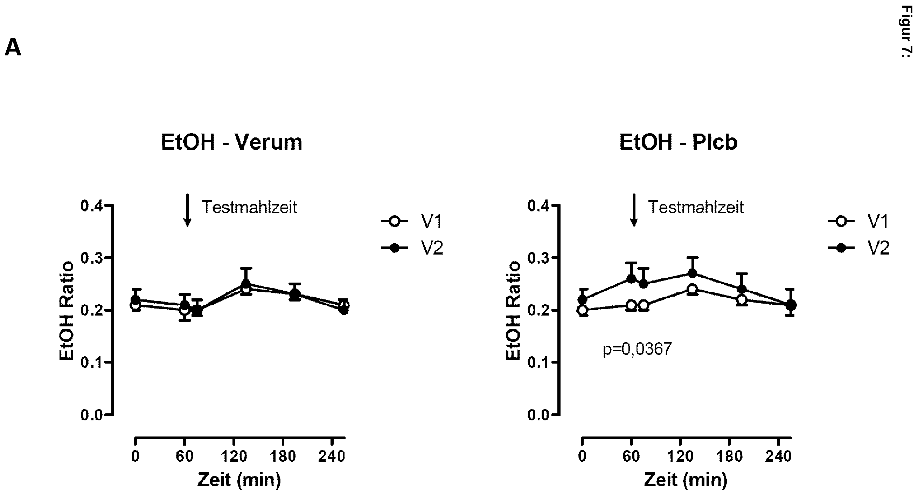

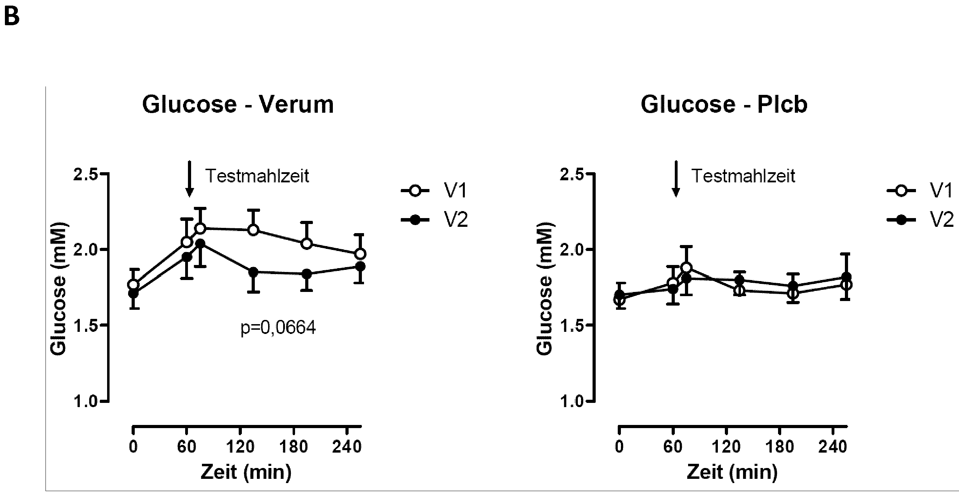

Figur 7 zeigt die basale und postprandiale EtOH-Ratio wie vorangehend definiert vor (V1) und nach (V2) Intervention (A) und die basale und postprandiale Dialysat-Glucose-Konzentration vor (V1) und nach (V2) Intervention (B).

In der Verum-Gruppe waren die Werte der EtOH-Ratio in V1 und V2 nahezu deckungsgleich, in der Placebo-Gruppe lagen die Werte in V2 höher als in V1. Dieser Unterschied war zwar mathematisch signifikant, klinisch aber unbedeutend und wurde nicht weiter berücksichtigt.

Postprandial stieg die Dialysat-Glucose im Muskel kurzzeitig an, und dieser Anstieg fiel in der Verum-Gruppe stärker aus als in der Placebo-Gruppe, sowohl in V1 als auch in V2. Möglicherweise hängt dieser Unterschied mit der Gruppenzusammensetzung zusammen: In der Verum-Gruppe war das Verhältnis Frauen/Männer gleich 17/3, in der Placebo-Gruppe 14/6. In der Verum-Gruppe fiel in V2 die Dialysat-Glucose schnell wieder ab. Da die Durchblutung, wie vorangehend bei der Diskussion der EtOH-Ratio beschrieben, eher unverändert war, ist davon auszugehen, dass Glucose unter Verum vermehrt in den Muskel aufgenommen und umgesetzt wurde.

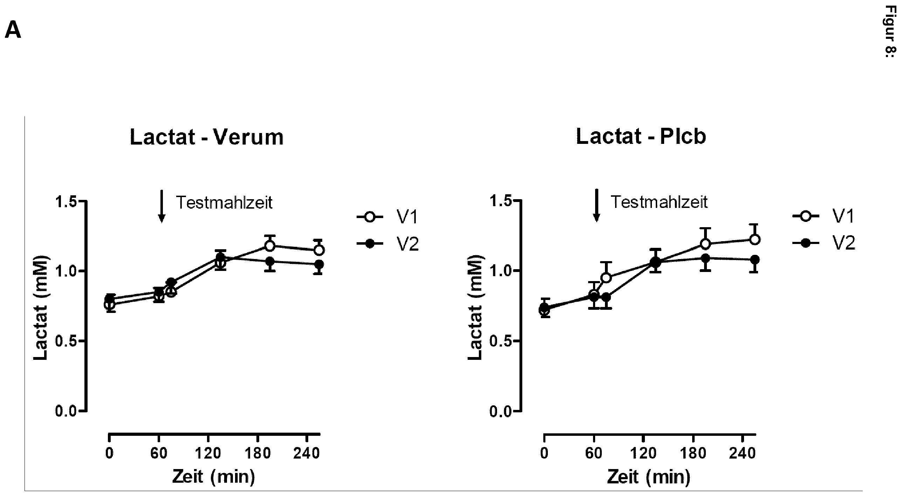

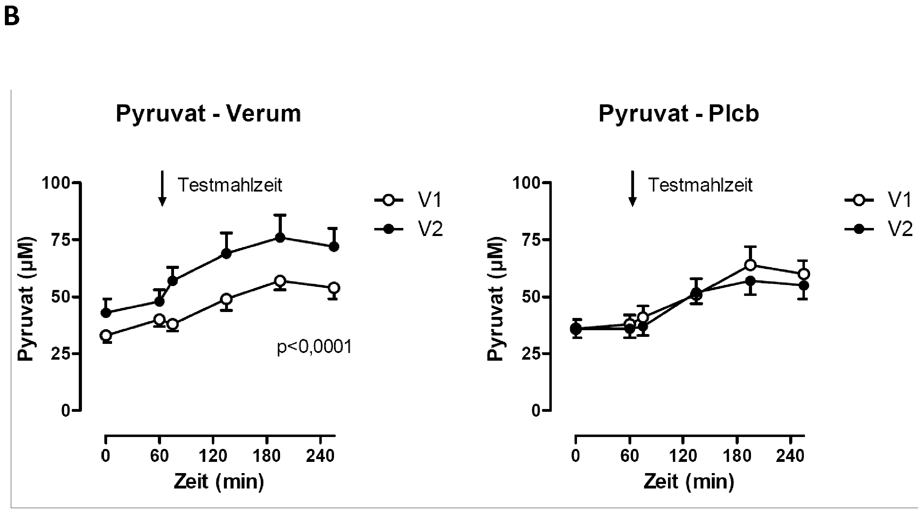

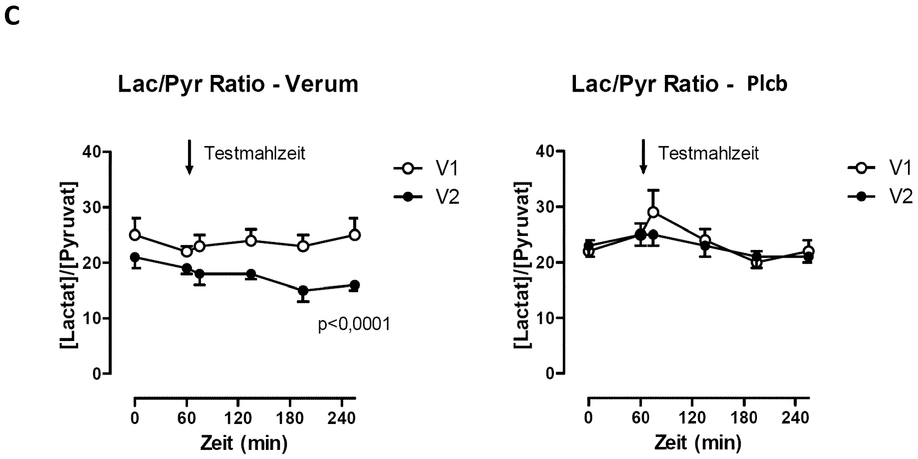

Figur 8 zeigt die basalen und postprandialen Dialysat-Lactat-Konzentrationen vor (V1) und nach (V2) Intervention (A), die basalen und postprandialen Pyruvat-Konzentrationen vor (V1) und nach (V2) Intervention (B) sowie die basale und postprandiale Lac/Pyr-Ratio vor (V1) und nach (V2) Intervention (C).

Wie vorangehend beschrieben, wird Glucose im Muskel zunächst zu Pyruvat und dann, je nach Sauerstoffangebot, entweder anaerob zu Lactat oder aerob zu Kohlendioxid und Wasser abgebaut. Postprandial stieg das Dialysat-Lactat sowohl in der Verum-Gruppe als auch in der Placebo-Gruppe auf das 1,5-fache - sowohl in V1 als auch in V2. Das Dialysat-Pyruvat stieg ebenso in beiden Gruppen und gleichermaßen in V1 und V2 auf das 2-fache. Jedoch waren in V2 der Verum-Gruppe sowohl die basalen als auch die postprandialen Pyruvat-Konzentrationen im Dialysat, wie vorangehend definiert, signifikant höher als in V1. Die Lactat/Pyruvat-Ratio änderte sich postprandial kaum in V1 und V2 der Placebo-Gruppe und V1 der Verum-Gruppe. Allerdings fiel sie deutlich ab in V2 der Verum-Gruppe. All diese Änderungen in V2 der Verum-Gruppe weisen auf einen deutlich verbesserten oxidativen Glucose-Stoffwechsel unter Verum-Supplementation hin.

Kurze Beschreibung der beigefügten Figuren:
Figur 1 zeigt eine Übersicht über den Ablauf eines Untersuchungstages: A) schematischer Ablauf der durchgeführten Tests, B) zeitlicher Ablauf der durchgeführten Tests an 40 Probanden.
Figur 2 zeigt: A) die basalen und postprandialen Blut-Glucose-Konzentrationen vor (V1) und nach (V2) Intervention und B) die basalen und postprandialen Insulin (B)-Konzentrationen vor (V1) und nach (V2) Intervention.

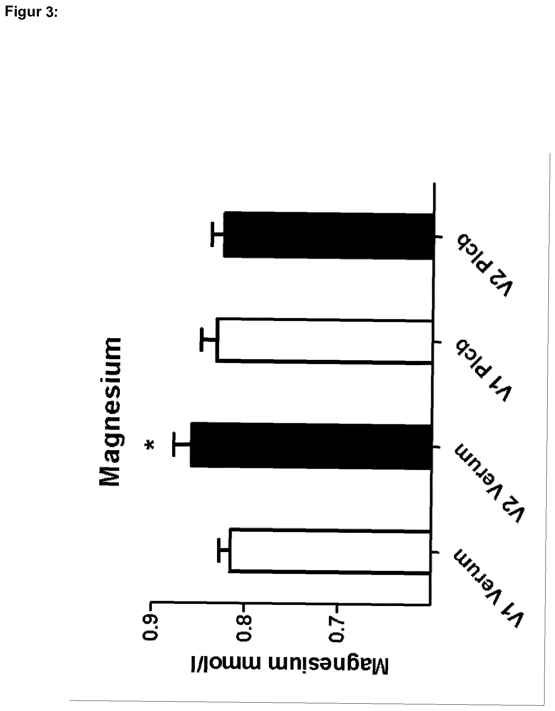

Figur 3 zeigt die basale Serum-Magnesium-Konzentrationen vor (V1) und nach (V2) Intervention.

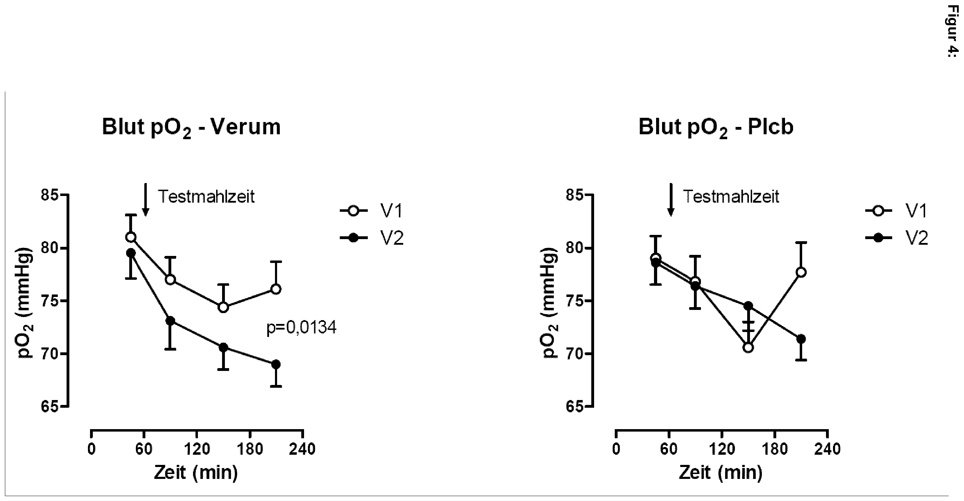

Figur 4 zeigt die basalen und postprandialen Blut-pO₂-Werte vor (V1) und nach (V2) Intervention.
Figur 5 zeigt die relativen Änderungen im Energieumsatz (EU) vor (V1) und nach (V2) Intervention.
Figur 6 zeigt die basale und postprandiale Kohlenhydratoxidationsrate (KHO) vor (V1) und nach (V2) Intervention.
Figur 7 zeigt: A) die basale und postprandiale EtOH-Ratio vor (V1) und nach (V2) Intervention und B) die basale und postprandiale Dialysat-Glucose-Konzentration vor (V1) und nach (V2) Intervention.
Figur 8 zeigt: A) die basalen und postprandialen Dialysat-Lactat-Konzentrationen vor (V1) und nach (V2) Intervention und B) die basalen und postprandialen Pyruvat-Konzentrationen vor (V1) und nach (V2) Intervention sowie C) die basale und postprandiale Lac/Pyr-Ratio vor (V1) und nach (V2) Intervention.

## Patentansprüche

1. Mischung aus drei oder mehr Citratsalzen, umfassend Magnesiumcitrat, wobei die Mischung aus drei oder mehr Citratsalzen zusätzlich Calciumcitrat und/oder Zinkcitrat umfasst, zur Anwendung in einem therapeutischen Verfahren zum Vorbeugen und/oder Behandeln einer Lactat-Azidose und/oder von Adipositas.

2. Mischung aus drei oder mehr Citratsalzen zur Anwendung gemäß Anspruch 1, wobei die Mischung mindestens Magnesiumcitrat, Calciumcitrat und Zinkcitrat umfasst oder daraus besteht.

3. Mischung aus drei oder mehr Citratsalzen zur Anwendung gemäß einem der vorangehenden Ansprüche, wobei, bezogen auf das Gesamtgewicht der Citratsalze, der Anteil an Magnesiumcitrat im Bereich von bis 2 bis 40 Gew.-% liegt, besonders bevorzugt im Bereich von 9 bis 36 Gew.-%, ganz besonders bevorzugt im Bereich von 15 bis 36 Gew.-%.

4. Zusammensetzung zur Anwendung in einem therapeutischen Verfahren, wobei die Zusammensetzung eine Mischung aus drei oder mehr Citratsalzen, umfassend Magnesiumcitrat, als aktiven Wirkstoff enthält, wobei die Mischung aus drei oder mehr Citratsalzen zusätzlich zum Magnesiumcitrat zwei oder mehr Citratsalze aus der Gruppe bestehend aus Calciumcitrat, Zinkcitrat, Natriumcitrat, Kupfercitrat, Eisencitrat, Kaliumcitrat, Kalium-Natrium-Hydrogencitrat, Calcium-Natrium-Hydrogencitrat, di-Natriumhydrogencitrat, Ammoniumcitrat, Ammoniumhydrogencitrat, Molybdäncitrat, Mangancitrat, Lithiumcitrat und Chromcitrat, umfasst, wobei das therapeutische Verfahren ein Verfahren zum Vorbeugen und/oder Behandeln einer Lactat-Azidose und/oder von Adipositas ist.

5. Zusammensetzung zur Anwendung in einem therapeutischen Verfahren gemäß Anspruch 4, wobei die Zusammensetzung zudem unabhängig voneinander einen oder mehrere weitere Bestandteile ausgewählt aus der Gruppe bestehend aus Süßungsmittel, vorzugsweise Laktose, Saccharose oder Sorbit; Säureregulatoren, vorzugsweise Kaliumtartrat; Trennmittel, vorzugsweise Salze, insbesondere Magnesium- oder Calciumsalze, von Speisefettsäuren, vernetzte Natriumcarboxymethylcellulose oder Siliciumdioxid; Säuerungsmittel, vorzugsweise Zitronensäure; Aromen, vorzugsweise natürliche Aromen; Füllstoffe, vorzugsweise Hydroxypropylcellulose; Magnesiumoxid; weitere Citratsalze, vorzugsweise Natriumcitrat, Kaliumcitrat, Kalium-Natrium-Hydrogencitrat, Calcium-Natrium-Hydrogencitrat, Kupfercitrat und/oder Eisencitrat; Calciumcarbonat, Calciumlactat, Magnesiumcarbonat und/oder Natriumhydrogencarbonat; Vitamine, vorzugsweise Riboflavin, Cholecalciferol (Vitamin D) und/oder Ascorbinsäure; L-Carnitin; Maltodextrin; Molybdän, vorzugsweise Natriummolybdat; Chrom, vorzugsweise Chromchlorid; und Selen, vorzugsweise Natriumselenit, Natriumselenat oder Selenhefe; umfasst.

6. Zusammensetzung zur Anwendung in einem therapeutischen Verfahren gemäß einem der Ansprüche 4 oder 5, wobei die Gesamtmenge an Citratsalz(en) in der Zusammensetzung, bezogen auf das Gesamtgewicht der Zusammensetzung, im Bereich von 4 bis 70 Gew.-% liegt, vorzugsweise im Bereich von 8 bis 67 Gew.-%, besonders bevorzugt im Bereich von 19 bis 44 Gew.-%, ganz besonders bevorzugt im Bereich von 25 bis 35 Gew.-%.

7. Zusammensetzung zur Anwendung in einem therapeutischen Verfahren gemäß einem der Ansprüche 4 bis 6, wobei die Zusammensetzung ausgewählt ist aus der Gruppe bestehend aus Granulaten, vorzugsweise zur Direkteinnahme, zum Auflösen oder zum Einrühren in Speisen, Tabletten, Kapseln und Dragees, vorzugsweise Tabletten, Kapseln und Dragees zum Schlucken, Lutschen oder Kauen, Pulver, vorzugsweise zur Direkteinnahme und wasserlösliche Pulver zum Trinken, Trinkampullen und Mikroperlen, vorzugsweise zur direkten Einnahme ohne Wasser.

8. Zusammensetzung zur Anwendung in einem therapeutischen Verfahren gemäß einem der Ansprüche 4 bis 7, wobei die Zusammensetzung frei von einem, mehreren oder sämtlichen der Bestandteile ausgewählt aus der Gruppe bestehend aus Laktose, Iod, Gluten, Aromastoffe und Süßungsmitteln ist, vorzugsweise frei ist von Laktose, Iod und Gluten.

9. Nicht-therapeutische Verwendung einer Mischung aus drei oder mehr Citratsalzen wie in einem der Ansprüche 1 bis 3 definiert oder einer Zusammensetzung wie in einem der Ansprüche 4 bis 8 definiert, zum Erreichen einer nicht-therapeutischen Wirkung ausgewählt aus der Gruppe bestehend aus: Verhinderung eines nicht-pathologischen Lactatüberschusses im Muskel durch das Anregen oder Verstärken des Kohlenhydratstoffwechsels, Förderung der Verdauung und einer unterstützenden Wirkung bei der Gewichtsreduktion.

## Claims

1. Mixture of three or more citrate salts, comprising magnesium citrate, wherein the mixture of three or more citrate salts additionally comprises calcium citrate and/or zinc citrate, for use in a therapeutic method for preventing and/or treating lactate acidosis and/or obesity.

2. Mixture of three or more citrate salts for use according to claim 1, wherein the mixture comprises or consists of at least magnesium citrate, calcium citrate and zinc citrate.

3. Mixture of three or more citrate salts for use according to any one of the preceding claims, wherein, based on the total weight of the citrate salts, the proportion of magnesium citrate is in the range of 2 to 40% by weight, particularly preferably in the range of 9 to 36% by weight, most preferably in the range of 15 to 36% by weight.

4. Composition for use in a therapeutic method, wherein the composition contains a mixture of three or more citrate salts, comprising magnesium citrate, as active substance, wherein the mixture of three or more citrate salts comprises additionally to magnesium citrate two or more citrate salts from the group consisting of calcium citrate, zinc citrate, sodium citrate, copper citrate, iron citrate, potassium citrate, potassium sodium hydrogen citrate, calcium sodium hydrogen citrate, disodium hydrogen citrate, ammonium citrate, ammonium hydrogen citrate, molybdenum citrate, manganese citrate, lithium citrate and chromium citrate, wherein the therapeutic method is a method for preventing and/or treating lactate acidosis and/or obesity.

5. Composition for use in a therapeutic method according to claim 4, wherein the composition further comprises, independently of each other, one or more further components selected from the group consisting of sweetener, preferably lactose, sucrose or sorbitol; acidity regulators, preferably potassium tartrate; separating agents, preferably salts, in particular magnesium or calcium salts, of fatty acids, crosslinked sodium carboxymethyl cellulose or silicon dioxide; acidifying agents, preferably citric acid; flavours, preferably natural flavours; fillers, preferably hydroxypropyl cellulose; magnesium oxide; other citrate salts, preferably sodium citrate, potassium citrate, potassium sodium hydrogen citrate, calcium sodium hydrogen citrate, copper citrate and/or iron citrate; calcium carbonate, calcium lactate, magnesium carbonate and/or sodium hydrogen carbonate; vitamins, preferably riboflavin, cholecalciferol (vitamin D) and/or ascorbic acid; L-carnitine; maltodextrin; molybdenum, preferably sodium molybdate; chromium, preferably chromium chloride; and selenium, preferably sodium selenite, sodium selenate or selenium yeast.

6. Composition for use in a therapeutic method according to one of claims 4 or 5, wherein the total amount of citrate salt(s) in the composition, based on the total weight of the composition, is in the range of 4 to 70% by weight, preferably in the range of 8 to 67% by weight, more preferably in the range of 19 to 44% by weight, most preferably in the range of 25 to 35% by weight.

7. Composition for use in a therapeutic method according to any one of claims 4 to 6, wherein the composition is selected from the group consisting of granules, preferably for direct consumption, for dissolving or for stirring into meals, tablets, capsules and lozenges, preferably tablets, capsules and lozenges for swallowing, sucking or chewing, powders, preferably for direct consumption and water-soluble powders for drinking, drinking vials and microbeads, preferably for direct consumption without water.

8. Composition for use in a therapeutic method according to any one of claims 4 to 7, wherein the composition is free of one, several or all of the components selected from the group consisting of lactose, iodine, gluten, flavouring agents and sweeteners, preferably free of lactose, iodine and gluten.

9. Non-therapeutic use of a mixture of three or more citrate salts as defined in any one of the claims 1 to 3 or of a composition as defined in any one of the claims 4 to 8 to achieve a non-therapeutic effect selected from the group consisting of: Preventing a non-pathological excess of lactate in the muscle by stimulating or enhancing the carbohydrate metabolism, promoting digestion and a supportive effect in weight loss.

## Revendications

1. Mélange de trois sels de citrate ou plus, comprenant du citrate de magnésium, dans lequel le mélange de trois sels de citrate ou plus comprend en outre du citrate de calcium et/ou du citrate de zinc, destiné à être utilisé dans un procédé thérapeutique pour prévenir et/ou pour traiter une acidose lactique et/ou l'obésité.

2. Mélange de trois sels de citrate ou plus destiné à être utilisé selon la revendication 1, dans lequel le mélange comprend au moins le citrate de magnésium, le citrate de calcium et le citrate de zinc ou consiste en ceux-ci.

3. Mélange de trois sels de citrate ou plus destiné à être utilisé selon l'une quelconque des revendications précédentes, dans lequel la proportion de citrate de magnésium, par rapport au poids total des sels de citrate, se situe dans la plage allant de 2 à 40 % en poids, de manière particulièrement préférée dans la plage allant de 9 à 36 % en poids, de manière tout particulièrement préférée dans la plage allant de 15 à 36 % en poids.

4. Composition destinée à être utilisée dans un procédé thérapeutique, dans lequel ladite composition comprend un mélange de trois sels de citrate ou plus, comprenant du citrate de magnésium, en tant que principe actif, dans lequel le mélange de trois sels de citrate ou plus comprend en plus du citrate de magnésium deux ou plusieurs sels de citrate du groupe consistant en citrate de calcium, en citrate de zinc, en citrate de sodium, en citrate de cuivre, en citrate de fer, en citrate de potassium, en citrate d'hydrogène de potassium et de sodium, en citrate d'hydrogène de sodium et de calcium, en citrate d'hydrogène disodique, en citrate d'ammonium, en citrate d'hydrogène d'ammonium, en citrate de molybdène, en citrate de manganèse, en citrate de lithium et en citrate de chrome, dans lequel le procédé thérapeutique est un procédé destiné à prévenir et/ou à traiter une acidose lactique et/ou l'obésité.

5. Composition destinée à être utilisée dans un procédé thérapeutique selon la revendication 4, dans laquelle la composition comprend en outre, indépendamment les uns des autres, un ou plusieurs autres composants choisis dans le groupe constitué par les édulcorants, de préférence le lactose, le saccharose ou le sorbitol; les correcteurs d'acidité, de préférence le tartrate de potassium; les anti-agglomérants, de préférence les sels, en particulier les sels de magnésium ou de calcium, les acides gras, la carboxyméthylcellulose de sodium réticulée ou le dioxyde de silicium; les acidifiants, de préférence l'acide citrique; les arômes, de préférence les arômes naturels; les charges, de préférence l'hydroxypropylcellulose; l'oxyde de magnésium; d'autres sels de citrate, de préférence le citrate de sodium, le citrate de potassium, le citrate d'hydrogène de potassium et de sodium, le citrate d'hydrogène de sodium et de calcium, le citrate de cuivre et/ou le citrate de fer; le carbonate de calcium, le lactate de calcium, le carbonate de magnésium et/ou le carbonate d'hydrogène de sodium; les vitamines, de préférence la riboflavine, le cholécalciférol (vitamine D) et/ou l'acide ascorbique; la L-carnitine; la maltodextrine; le molybdène, de préférence le molybdate de sodium; le chrome, de préférence le chlorure de chrome; et le sélénium, de préférence le sélénite de sodium, le sélénate de sodium ou la levure de sélénium.

6. Composition destinée à être utilisée dans un procédé thérapeutique selon l'une quelconque des revendications 4 ou 5, dans laquelle la quantité totale de sel(s) de citrate dans la composition, par rapport au poids total de la composition, se situe dans la plage allant de 4 à 70 % en poids, de préférence dans la plage allant de 8 à 67 % en poids, de manière particulièrement préférée dans la plage allant 19 à 44 % en poids, de manière tout particulièrement préférée dans la plage allant de 25 à 35 % en poids.

7. Composition destinée à être utilisée dans un procédé thérapeutique selon l'une quelconque des revendications 4 à 6, dans laquelle la composition est choisie dans le groupe constitué par les granulés, de préférence pour l'ingestion directe, pour la dissolution ou pour les ajouter en remuant dans des plats, les comprimés, les capsules et les dragées, de préférence les comprimés, les capsules et les dragées pour avaler, sucer ou mâcher, les poudres, de préférence pour l'ingestion directe, et les poudres hydrosolubles pour boire, les ampoules à boire et les microbilles, de préférence pour l'ingestion directe sans eau.

8. Composition destinée à être utilisée dans un procédé thérapeutique selon l'une quelconque des revendications 4 à 7, dans laquelle la composition est exempte d'un, de plusieurs ou de tous les ingrédients choisis dans le groupe constitué par le lactose, l'iode, le gluten, les arômes et les édulcorants, de préférence est exempte de lactose, d'iode et de gluten.

9. Utilisation non thérapeutique d'un mélange de trois sels de citrate ou plus, tel que défini dans l'une quelconque des revendications 1 à 3, ou d'une composition telle que définie dans l'une quelconque des revendications 4 à 8, pour obtenir un effet non thérapeutique choisi dans le groupe constitué par: la prévention d'un excès non pathologique de lactate dans le muscle en stimulant ou en renforçant le métabolisme des glucides, la facilitation de la digestion et un effet supportant dans la réduction de poids.
